# EUROPEAN PATENT APPLICATION

(11) **EP 4 265 614 A1**
(43) Date of publication of application: **25.10.2023**
(21) Application number: 21906706.3
(22) Date of filing: 16.12.2021
(51) Int. Cl.: C07D 487/04, C08F 38/02, C08G 65/34, C08G 83/00

(54) **COMPOUND AND COMPOSITION**

(30) Priority: 17.12.2020 JP 2020209158; 17.12.2020 JP 2020209155
(71) Applicant: ADEKA CORPORATION, Arakawa-ku Tokyo 116-8554 (JP)
(72) Inventor: SAITO, Hiromasa, Tokyo 116-8554 (JP); MURAMATSU, Yousuke, Tokyo 116-8554 (JP); SUZUKI, Mizuki, Tokyo 116-8554 (JP); IRISAWA, Masatomi, Tokyo 116-8554 (JP)
(74) Representative: Forstmeyer, Dietmar
(86) International application number: PCT/JP2021/046616
(87) International publication number: WO 2022/131346

(57) **Abstract**

An object of the present invention is to provide a composition that gives a cured product excellent in heat resistance and solvent resistance. The present invention provides a compound represented by the general formula (I) below and having at least one reactive group in a molecule. In the formula, A represents a hydrocarbon ring having 6 carbon atoms; X¹ and X² each represent, for example, an aryl group having 6 to 30 carbon atoms and optionally substituted with a reactive group or a group having a reactive group; R¹, R², R³, R⁴, R⁶, R⁷, R⁸, and R⁹ each represent, for example, a hydrogen atom, a reactive group, or a hydrocarbon group having 1 to 20 carbon atoms and optionally substituted with a reactive group; and R⁵ and R¹⁰ each represent, for example, a hydrogen atom, or a hydrocarbon group having 1 to 20 carbon atoms and optionally substituted with a reactive group.

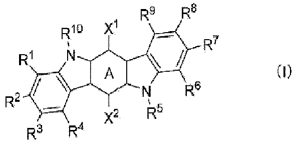

## Description

### Technical Field

The present invention relates to a compound having a specific skeleton.

### Background Art

Various materials typified epoxy resins are used as resin materials for electronic components used in semiconductor devices, printed circuit boards, and the like. Recently, however, materials having excellent heat resistance have been in particular demand.

For example, Patent Literature 1 discloses a heat-curable composition containing a phenolic compound and an epoxy compound. Patent Literature 2 discloses a vinylbenzyl-modified monofunctional phenolic compound and an active ester resin formed by using the vinylbenzyl-modified monofunctional phenolic compound, and purports that the active ester resin can form a cured product having excellent dielectric properties and excellent heat resistance.

Patent Literature 3 discloses a curable composition containing a compound having an indolocarbazole skeleton with a polymerizable group and a crosslinkable compound having two polymerizable groups.

Patent Literature 4 discloses an oxazine compound having a group having a carbon-carbon triple bond structure, and purports that the compound that has excellent heat resistance, causes small thermal expansion, and is also excellent in adhesion and moisture and solder resistance.

### Citation List

### Patent Literature

Patent Literature 1: WO 2019/203292
Patent Literature 2: WO 2020/059624
Patent Literature 3: US 9614172
Patent Literature 4: JP 2018-002612A

### Summary of Invention

An object of the present invention is to provide a composition that gives a cured product excellent in heat resistance and excellent solvent resistance.

As a result of in-depth research, the inventors of the present invention have found that a composition containing a compound having a specific skeleton and having at least one reactive group in a molecule gives a cured product excellent in heat resistance and solvent resistance, and have thus accomplished the present invention.

Specifically, the present invention provides a compound represented by the general formula (I) below and having at least one reactive group in a molecule (hereinafter also referred to as "compound (I)"). 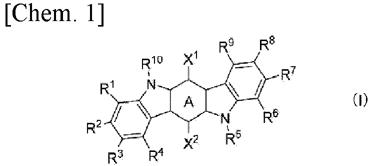
In the formula, A represents a hydrocarbon ring having 6 carbon atoms;
X¹ and X² each independently represent an aryl group having 6 to 30 carbon atoms and optionally substituted with a reactive group or a group having a reactive group, a heterocyclic group having 2 to 30 carbon atoms and optionally substituted with a reactive group or a group having a reactive group, or a heterocycle-containing group having 3 to 30 carbon atoms and optionally substituted with a reactive group or a group having a reactive group;
R¹, R², R³, R⁴, R⁶, R⁷, R⁸, and R⁹ each independently represent a hydrogen atom, a halogen atom, a reactive group, a nitro group, an aliphatic hydrocarbon group having 1 to 20 carbon atoms and optionally substituted with a reactive group, an aromatic hydrocarbon ring-containing group having 6 to 20 carbon atoms and optionally substituted with a reactive group, a heterocyclic group having 2 to 10 carbon atoms and optionally substituted with a reactive group, or a heterocycle-containing group having 3 to 30 carbon atoms and optionally substituted with a reactive group, or a group formed by replacing at least one methylene group in the aliphatic hydrocarbon group having 1 to 20 carbon atoms by a divalent group selected from <group A> below, a group formed by replacing at least one methylene group in the aromatic hydrocarbon ring-containing group having 6 to 20 carbon atoms by a divalent group selected from <group A> below, or a group formed by replacing at least one methylene group in the heterocycle-containing group having 3 to 30 carbon atoms by a divalent group selected from <group A> below; and
R⁵ and R¹⁰ each independently represent a hydrogen atom, an aliphatic hydrocarbon group having 1 to 20 carbon atoms and optionally substituted with a reactive group, an aromatic hydrocarbon ring-containing group having 6 to 20 carbon atoms and optionally substituted with a reactive group, a heterocyclic group having 2 to 10 carbon atoms and optionally substituted with a reactive group, or a heterocycle-containing group having 3 to 30 carbon atoms and optionally substituted with a reactive group, or a group formed by replacing at least one methylene group in the aliphatic hydrocarbon group having 1 to 20 carbon atoms by a divalent group selected from <group A> below, a group formed by replacing at least one methylene group in the aromatic hydrocarbon ring-containing group having 6 to 20 carbon atoms by a divalent group selected from <group A> below, or a group formed by replacing at least one methylene group in the heterocycle-containing group having 3 to 30 carbon atoms by a divalent group selected from <group A> below:
   <group A>: -O-, -CO-, -COO-, -OCO-, -NR¹¹-, -NR¹²CO-, -S-
   R¹¹ and R¹² each independently represent a hydrogen atom or a hydrocarbon group having 1 to 20 carbon atoms; and
   with a proviso that the reactive group above is a group selected from a carbon-carbon double bond, a carbon-carbon triple bond, a nitrile group, an epoxy group, an isocyanate group, a hydroxy group, a phenolic hydroxy group, an amino group, and a thiol group.

Also, the present invention provides a composition containing the compound (I).

### Description of Embodiments

The compound of the present invention is a compound having a specific skeleton and represented by the general formula (I) above, and has at least one reactive group in a molecule.

The term "reactive group" as used for the compound of the present invention means a group that is reactive with another reactive group to form a covalent bond. Here, the other reactive group may be the same reactive group or a different reactive group. The term "reactive group" as used in the present invention means a carbon-carbon double bond (vinyl group), a carbon-carbon triple bond (ethynyl group), a nitrile group, an epoxy group, an isocyanate group, a hydroxy group, a phenolic hydroxy group, an amino group, and a thiol group. That is to say, a reactive group in the general formula (I) above is a vinyl group, an ethynyl group, a nitrile group, an epoxy group, an isocyanate group, a hydroxy group, a phenolic hydroxy group, an amino group, or a thiol group. A nitrile group can form a triazine ring through a trimerization reaction and is therefore encompassed by the reactive group. As the reactive group, a carbon-carbon triple bond, a carbon-carbon double bond, a phenolic hydroxy group, and an epoxy group are preferable, a carbon-carbon triple bond, a carbon-carbon double bond, and a phenolic hydroxy group are more preferable, and a carbon-carbon triple bond and a phenolic hydroxy group are particularly preferable. The reason for this is that a cured product having good heat resistance can be obtained when the reactive group is any of these groups.

Examples of the group having a reactive group in the general formula (I) include an alkenyl group having 2 to 20 carbon atoms, such as an allyl group; an alkenyloxy group having 2 to 20 carbon atoms, such as a vinyloxy group and an allyloxy group; an alkynyl group having 2 to 20 carbon atoms, such as a propargyl group; an alkynyloxy group having 2 to 20 carbon atoms, such as a propargyloxy group; and an acrylate group, a methacrylate group, a hydroxyphenyl group, a hydroxynaphthyl group, a glycidyl group, and a glycidyloxy group.

The alkenyl group having 2 to 20 carbon atoms may be linear or branched. The alkenyl group having 2 to 20 carbon atoms may be a terminal alkenyl group, which has an unsaturated bond at the terminal, or an internal alkenyl group, which has an internal unsaturated bond. Examples of the terminal alkenyl group include vinyl, allyl, 2-methyl-2-propenyl, 3-butenyl, 4-pentenyl, and 5-hexenyl. Examples of the internal alkenyl group include 2-butenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, and 4,8,12-tetradecatrienylallyl.

The alkynyl group having 2 to 20 carbon atoms may be linear or branched. The alkynyl group having 2 to 20 carbon atoms may be a terminal alkynyl group, which has an unsaturated bond at the terminal, or an internal alkynyl group, which has an internal unsaturated bond. Examples of the terminal alkynyl group include ethynyl, propargyl, 2-methyl-2-propynyl, 3-butynyl, 4-pentynyl, and 5-hexynyl. Examples of the internal alkynyl group include 2-butynyl, 3-pentynyl, 2-hexynyl, 3-hexynyl, 2-heptynyl, 3-heptynyl, 4-heptynyl, 3-octynyl, 3-nonynyl, 4-decynyl, 3-undecynyl, and 4-dodecynyl.

An example of the alkenyloxy group having 2 to 20 carbon atoms is a group in which the alkenyl group having 2 to 20 carbon atoms is bonded to an oxygen atom. An example of the alkynyloxy group having 2 to 20 carbon atoms is a group in which the alkynyl group having 2 to 20 carbon atoms is bonded to an oxygen atom.

Examples of the hydrocarbon ring having 6 carbon atoms represented by A in the general formula (I) include a benzene ring, a cyclohexadiene ring, a cyclohexene ring, and a cyclohexane ring.

The aryl group having 6 to 30 carbon atoms represented by X¹ and X² in the general formula (I) may have a monocyclic structure, a fused ring structure, or a structure in which two aromatic hydrocarbon rings are linked together. The aryl group having 6 to 30 carbon atoms and having a fused ring structure may be a hydrocarbon-type aromatic fused ring group having 7 to 30 carbon atoms and having a structure in which two or more aromatic hydrocarbon rings are fused together.

Examples of the aryl group having 6 to 30 carbon atoms and having a monocyclic structure include phenyl, tolyl, xylyl, ethylphenyl, and 2,4,6-trimethylphenyl. Examples of the hydrocarbon-type aromatic fused ring group having 7 to 30 carbon atoms include naphthyl, anthracenyl, phenanthryl, pyrenyl, fluorenyl, and indenofluorenyl.

The aryl group in which two aromatic hydrocarbon rings are linked together may be an aryl group in which two monocyclic aromatic hydrocarbon rings are linked together, an aryl group in which a monocyclic aromatic hydrocarbon ring and an aromatic hydrocarbon ring having a fused ring structure are linked together, or an aryl group in which an aromatic hydrocarbon ring having a fused ring structure and another aromatic hydrocarbon ring having a fused ring structure are linked together.

Examples of a linking group for linking two aromatic hydrocarbon rings include a single bond, a sulfide group (-S-), and a carbonyl group.

Examples of the aryl group in which two monocyclic aromatic hydrocarbon rings are linked together include biphenyl, diphenylsulfide, and benzoylphenyl.

The aryl group having 6 to 30 carbon atoms may be substituted with a reactive group or a group having a reactive group. An aryl group having 6 to 30 carbon atoms and substituted with a reactive group or a group having a reactive group means a group having 6 to 30 carbon atoms formed by replacing at least one hydrogen atom in the aryl group by a reactive group or a group having a reactive group. The aryl group having 6 to 30 carbon atoms may also have another substituent. Examples of the other substituent include a halogen atom and a nitro group.

The aryl group having 6 to 30 carbon atoms may be substituted with a phenolic hydroxy group. An aryl group having 6 to 30 carbon atoms and substituted with a phenolic hydroxy group means a group having 6 to 30 carbon atoms formed by replacing at least one hydrogen atom in an aromatic hydrocarbon ring of the aryl group by a hydroxy group. The aryl group having 6 to 30 carbon atoms may have a substituent other than a phenolic hydroxy group. Examples of the substituent include a halogen atom, a nitrile group, a nitro group, an amino group, a carboxyl group, a methacryloyl group, an acryloyl group, an epoxy group, a vinyl group, a vinyl ether group, a mercapto group, and an isocyanate group.

The number of carbon atoms specified in the present invention includes the number of carbon atoms in a substituent.

For example, a methylphenyl group is an aryl group having 7 carbon atoms.

A heterocyclic group having 2 to 30 carbon atoms represented by X¹ and X² in the general formula (I) refers to a group obtained by removing one hydrogen atom from a heterocyclic compound; however, an epoxy group is not encompassed by the heterocyclic group since it is encompassed by the reactive group. The heterocyclic group may have a monocyclic structure or a fused ring structure. The heterocyclic group having 2 to 30 carbon atoms and having a fused ring structure may be, for example, a heterocycle-containing fused ring group having 3 to 30 carbon atoms and having a structure in which a heterocycle and another heterocycle or a hydrocarbon ring are fused together. Specific examples of the heterocyclic group include pyridyl, quinolyl, thiazolyl, tetrahydrofuranyl, dioxolanyl, tetrahydropyranyl, methylthiophenyl, hexylthiophenyl, benzothiophenyl, pyrrolyl, pyrrolidinyl, imidazolyl, imidazolidinyl, imidazolinyl, pyrazolyl, pyrazolidinyl, piperidinyl, piperazinyl, pyrimidinyl, furyl, thienyl, benzoxazol-2-yl, thiazolyl, isothiazolyl, oxazolyl, isoxazolyl, and morpholnyl.

The heterocyclic group having 2 to 30 carbon atoms may be substituted with a reactive group or a group having a reactive group. A heterocyclic group having 2 to 30 carbon atoms and substituted with a reactive group or a group having a reactive group means a group having 2 to 30 carbon atoms formed by replacing at least one hydrogen atom in the heterocyclic group by a reactive group or a group having a reactive group. The heterocyclic group having 2 to 30 carbon atoms may have another substituent. Examples of the substituent include a halogen atom and a nitro group.

Examples of the heterocyclic group having 2 to 30 carbon atoms also include a heteroaryl group having 3 to 30 carbon atoms. The heteroaryl group may have a monocyclic structure of an aromatic heterocyclic ring, a fused ring structure composed of an aromatic hydrocarbon ring and an aromatic heterocyclic ring, or a fused ring structure composed of two aromatic heterocyclic rings.

Examples of the heteroaryl group having a monocyclic structure include pyrrolyl, pyridyl, furyl, thienyl, imidazolyl, oxazolyl, thiazolyl, and triazinyl.

Examples of the heteroaryl group having a fused ring structure include benzofuranyl, dibenzofuranyl, indolyl, carbazolyl, quinolyl, naphthyridinyl, benzoimidazolyl, benzoxazolyl, and benzothiophenyl.

The heteroaryl group having 3 to 30 carbon atoms may be substituted with a phenolic hydroxy group. A heteroaryl group having a phenolic hydroxy group means a group having 8 to 30 carbon atoms formed by replacing at least one hydrogen atom in an aromatic hydrocarbon ring of the heteroaryl group by a hydroxy group. The heteroaryl group having 3 to 30 carbon atoms may have a substituent other than a phenolic hydroxy group. Examples of the substituent include a halogen atom, a nitrile group, a nitro group, an amino group, a carboxyl group, a methacryloyl group, an acryloyl group, an epoxy group, a vinyl group, a vinyl ether group, a mercapto group, and an isocyanate group.

The heterocycle-containing group having 3 to 30 carbon atoms represented by X¹ and X² in the general formula (I) means a group having 3 to 30 carbon atoms formed by replacing at least one hydrogen atom in a hydrocarbon group by a heterocyclic group. Examples of the heterocyclic group include the groups listed above as examples of the heterocyclic group having 2 to 30 carbon atoms. The hydrocarbon group may be a hydrocarbon group having 1 to 20 carbon atoms. The hydrocarbon group having 1 to 20 carbon atoms will be described later.

The heterocycle-containing group having 3 to 30 carbon atoms may be substituted with a reactive group or a group having a reactive group. A heterocycle-containing group having 3 to 30 carbon atoms and substituted with a reactive group or a group having a reactive group means a group having 3 to 30 carbon atoms formed by replacing at least one hydrogen atom in the heterocycle-containing group by a reactive group or a group having a reactive group. The heterocycle-containing group having 3 to 30 carbon atoms may have another substituent. Examples of the substituent include a halogen atom and a nitro group.

The aliphatic hydrocarbon group having 1 to 20 carbon atoms represented by R¹, R², R³, R⁴, R⁵, R⁶, R⁷, R⁸, R⁹, and R¹⁰ (hereinafter referred to as "R¹ etc.") in the general formula (I) refers to a group that is composed of carbon and hydrogen atoms, has 1 to 20 carbon atoms, and contains no aromatic hydrocarbon ring. The aromatic hydrocarbon ring-containing group having 6 to 20 carbon atoms refers to a group that is composed of carbon and hydrogen atoms, has 6 to 20 carbon atoms, and contains an aromatic hydrocarbon ring. However, a reactive group or a group having a reactive group, that is, a vinyl group, an ethynyl group, and groups having any of these groups are not encompassed by the above-described hydrocarbon group.

Examples of the aliphatic hydrocarbon group having 1 to 20 carbon atoms include an alkyl group having 1 to 20 carbon atoms, a cycloalkyl group having 3 to 20 carbon atoms, and a cycloalkylalkyl group having 4 to 20 carbon atoms.

The alkyl group having 1 to 20 carbon atoms may be linear or branched. Examples of the linear alkyl group include methyl, ethyl, propyl, butyl, iso-amyl, tert-amyl, hexyl, heptyl, and octyl. Examples of the branched alkyl group include iso-propyl, sec-butyl, tert-butyl, iso-butyl, iso-pentyl, tert-pentyl, 2-hexyl, 3-hexyl, 2-heptyl, 3-heptyl, iso-heptyl, tert-heptyl, iso-octyl, tert- octyl, 2-ethylhexyl, nonyl, isononyl, decyl, dodecyl, tridecyl, tetradecyl, pentadecyl, hexadecyl, hebrotadecyl, and octadecyl.

Examples of the cycloalkyl group having 3 to 20 carbon atoms include a saturated monocyclic alkyl group having 3 to 20 carbon atoms, a saturated polycyclic alkyl group having 3 to 20 carbon atoms, and a group having 4 to 20 carbon atoms and formed by replacing at least one hydrogen atom in a ring of the saturated monocyclic or polycyclic alkyl group by an alkyl group. Examples of the saturated monocyclic alkyl group include cyclopropyl, cyclobutyl, cyclopentyl, cyclohexyl, cycloheptyl, cyclooctyl, cyclononyl, and cyclodecyl. Examples of the saturated polycyclic alkyl group include adamantyl, decahydronaphthyl, octahydropentalene, and bicyclo[1.1.1]pentanyl. Examples of the alkyl group for replacing at least one hydrogen atom in a ring of the saturated monocyclic or saturated polycyclic alkyl group include the groups listed above as examples of the alkyl group having 1 to 20 carbon atoms. Examples of the group formed by replacing at least one hydrogen atom in a ring of a saturated polycyclic alkyl group by an alkyl group include bornyl.

The cycloalkylalkyl group having 4 to 20 carbon atoms means a group having 4 to 20 carbon atoms and formed by replacing a hydrogen atom in an alkyl group by a cycloalkylalkyl group. The cycloalkyl group in the cycloalkylalkyl group may be monocyclic or polycyclic. Examples of the cycloalkylalkyl group having 4 to 20 carbon atoms in which the cycloalkyl group is monocyclic include cyclopropylmethyl, 2-cyclobutylethyl, 3-cyclopentylpropyl, 4-cyclohexylbutyl, cycloheptylmethyl, cyclooctylmethyl, 2-cyclononylethyl, and 2-cyclodecylethyl. Examples of the cycloalkylalkyl group having 4 to 20 carbon atoms in which the cycloalkyl group is polycyclic include 3-3-adamantylpropyl and decahydronaphthylpropyl.

The aromatic hydrocarbon ring-containing group having 6 to 20 carbon atoms is a hydrocarbon group containing an aromatic hydrocarbon ring and no heterocyclic ring and may have an aliphatic hydrocarbon group. Examples of the aromatic hydrocarbon ring-containing group include an aryl group having 6 to 20 carbon atoms and an arylalkyl group having 7 to 20 carbon atoms.

Examples of the aryl group having 6 to 20 carbon atoms include the groups listed above as examples of the aryl group having 6 to 30 carbon atoms represented by X¹ and X².

The arylalkyl group having 7 to 20 carbon atoms means a group formed by replacing at least one hydrogen atom in an alkyl group by an aryl group. Examples of the arylalkyl group having 7 to 20 carbon atoms include benzyl, fluorenyl, indenyl, 9-fluorenylmethyl, α-methylbenzyl, α,α-dimethylbenzyl, phenylethyl, and naphthylpropyl.

The aliphatic hydrocarbon group having 1 to 20 carbon atoms represented by R¹ etc. in the general formula (I) and the aromatic hydrocarbon ring-containing group having 6 to 20 carbon atoms represented by R¹ etc. in the general formula (I) may have a substituent. Examples of the substituent include a halogen atom and a nitro group. When the aliphatic hydrocarbon group or the aromatic hydrocarbon ring-containing group has a substituent, the number of carbon atoms indicates the number of carbon atoms in the entire group.

R¹ etc. in the general formula (I) each may be a group formed by replacing at least one methylene group in the aliphatic hydrocarbon group having 1 to 20 carbon atoms by a divalent group selected from <group A>, a group formed by replacing at least one methylene group in the aromatic hydrocarbon ring-containing group having 6 to 20 carbon atoms by a divalent group selected from <group A>, or a group formed by replacing at least one methylene group in the heterocycle-containing group having 3 to 30 carbon atoms by a divalent group selected from <group A>. The number of carbon atoms in these groups indicates the number of carbon atoms in the aliphatic hydrocarbon group, the aromatic hydrocarbon ring-containing group, or the heterocycle-containing group before replacing at least one the methylene group thereof.

The aliphatic hydrocarbon group having 1 to 20 carbon atoms represented by R¹¹ and R¹² in the general formula (I) and the aromatic hydrocarbon ring-containing group having 6 to 20 carbon atoms represented by R¹¹ and R¹² in the general formula (I) are the same as described for the aliphatic hydrocarbon group having 1 to 20 carbon atoms represented by R¹ etc. in the general formula (I) and the aromatic hydrocarbon ring-containing group having 6 to 20 carbon atoms represented by R¹ etc. in the general formula (I).

In the present invention, a compound in which A in the general formula (I) is a benzene ring, a cyclohexadiene ring, or a cyclohexane ring is preferable because it has excellent solubility in solvents and provides a cured product excellent in heat resistance and solvent resistance. That is to say, the compound (I) is preferably a compound represented by the following general formula (Ia), (Ib), or (Ic).

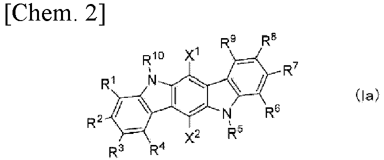

The symbols in the formula are as defined in the general formula (I).

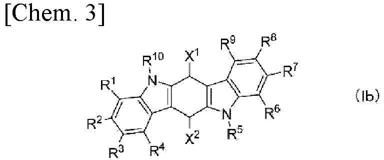

The symbols in the formula are as defined in the general formula (I).

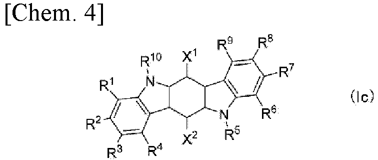

The symbols in the formula are as defined in the general formula (I).

In particular, a compound represented by the general formula (Ia), in which A in the general formula (I) is a benzene ring, is preferable because it provides a cured product even more excellent in heat resistance and solvent resistance.

When the reactive group is a group other than a phenolic hydroxy group, a compound in which X¹ and X² in the general formula (I) each represent an aryl group having 6 to 30 carbon atoms and having a monocyclic structure, a hydrocarbon-type aromatic fused ring group having 7 to 30 carbon atoms, or a heterocycle-containing fused ring group having 3 to 30 carbon atoms can provide a cured product having even more excellent heat resistance and is thus preferable. As the aryl group having 6 to 30 carbon atoms and having a monocyclic structure, a phenyl group is particularly preferable in view of high solubility in solvents.

The hydrocarbon-type aromatic fused ring group having 7 to 30 carbon atoms is a fused ring containing an aromatic ring consisting of carbon and hydrogen atoms, wherein only the carbon atoms constitute the ring structure of the fused ring. Examples of the hydrocarbon-type aromatic fused ring having 7 to 30 carbon atoms include a naphthyl group, an anthracenyl group, a pyrenyl group, a tetracenyl group, a triphenylenyl group, an azulenyl group, a phenanthrenyl group, a tetracenyl group, a perylenyl group, and a fluorenyl group. A naphthyl group and a fluorenyl group are preferred in view of high solubility in solvents.

The heterocycle-containing fused ring group having 3 to 30 carbon atoms is a fused ring having 3 to 30 carbon atoms and containing a heterocyclic ring. Examples of the heterocycle-containing fused ring group having 3 to 30 carbon atoms include an indolyl group, a carbazolyl group, a benzofuranyl group, a benzothiophenyl group, a benzopyrazoyl group, a julolidinyl group, and a benzoquinolinyl group. An indolyl group, a carbazolyl group, and a benzothiophenyl group are preferred in view of high solubility in solvents.

When the reactive group is a group other than a phenolic hydroxy group, a compound in which X¹ and X² in the general formula (I) are each independently a group optionally substituted with a reactive group or a group having a reactive group can provide a cured product having excellent heat resistance, and is therefore preferred.

When X¹ or X² is an aryl group having 6 to 30 carbon atoms and substituted with a reactive group or a group having a reactive group, the number of substituents is preferably 1 or 2.

When X¹ and X² have a reactive group or a group having a reactive group, the reactive group is preferably an ethynyl group, in view of obtaining a cured product having excellent heat resistance. As the group having an ethynyl group, an alkynyl group having 2 to 10 carbon atoms and an alkynyloxy group having 2 to 10 carbon atoms are preferable, and a propargyl group and a propargyloxy group are more preferable.

X¹ and X² may be the same group or different groups, but are preferably the same group in view of synthesis of the compound.

When the reactive group is a group other than a phenolic hydroxy group, R⁵ and R¹⁰ in the general formula (I) are each preferably a hydrocarbon group having 1 to 20 carbon atoms and substituted with a reactive group. When X⁵ and X¹⁰ are groups having a reactive group, the reactive group is preferably an ethynyl group or a vinyl group, and particularly preferably an ethynyl group, in view of obtaining a cured product having excellent heat resistance. As the hydrocarbon group having 1 to 20 carbon atoms and substituted with an ethynyl group, an alkynyl group having 3 to 10 is preferable, and a propargyl group is more preferable.

R¹, R², R³, R⁴, R⁶, R⁷, R⁸, and R⁹ are preferably hydrogen atoms in view of synthesis of the compound.

When the reactive group is a group other than a phenolic hydroxy group, the compound preferably has three or more reactive groups in the molecule, in view of obtaining a cured product having excellent heat resistance, and particularly preferably, the compound has four to six reactive groups.

Examples of the case in which the compound has three or more reactive groups in the molecule include: a case in which X¹ and X² each have two reactive groups; a case in which X¹ and X² each have two reactive groups and, furthermore, R⁵ and R¹⁰ have a reactive group; and a case in which X¹ and X² each have a single reactive group and, furthermore, R⁵ and R¹⁰ have a reactive group.

When the reactive group is a phenolic hydroxy group, at least one of X¹ and X² in the general formula (I) is preferably a phenyl group, a biphenyl group, a naphthyl group, an anthracenyl group, a pyrenyl group, or a fluorenyl group each optionally substituted with a phenolic hydroxy group, in view of obtaining a cured product having excellent heat resistance. Preferably, both X¹ and X² are groups selected from the group consisting of a phenyl group, a naphthyl group, an anthracenyl group, and a pyrenyl group each having a phenolic hydroxy group, and particularly preferably, only both X¹ and X² are groups selected from the group consisting of a phenyl group, a naphthyl group, an anthracenyl group, and a pyrenyl group each having a phenolic hydroxy group. As X¹ and X², a phenyl or naphthyl group having a phenolic hydroxy group is particularly preferable, in view of obtaining a cured product having excellent heat resistance.

X¹ and X² may be the same group or different groups, but are preferably the same group in view of synthesis of the compound.

R¹, R², R³, R⁴, R⁶, R⁷, R⁸, and R⁹ are preferably hydrogen atoms in view of synthesis of the compound.

R⁵ and R¹⁰ are each preferably a hydrogen atom or an alkyl group having 1 to 4 carbon atoms, and particularly preferably a hydrogen atom, in view of obtaining a cured product having excellent heat resistance.

When the reactive group is a group other than a phenolic hydroxy group, the compound preferably has two or more phenolic hydroxy groups in the molecule, in view of obtaining a cured product having excellent heat resistance. The compound more preferably has four or more phenolic hydroxy groups, and particularly preferably has four to six phenolic hydroxy groups.

Specific examples of the compound (I) in which the reactive group is a group other than a phenolic hydroxy group include the following compounds (1) to (133).

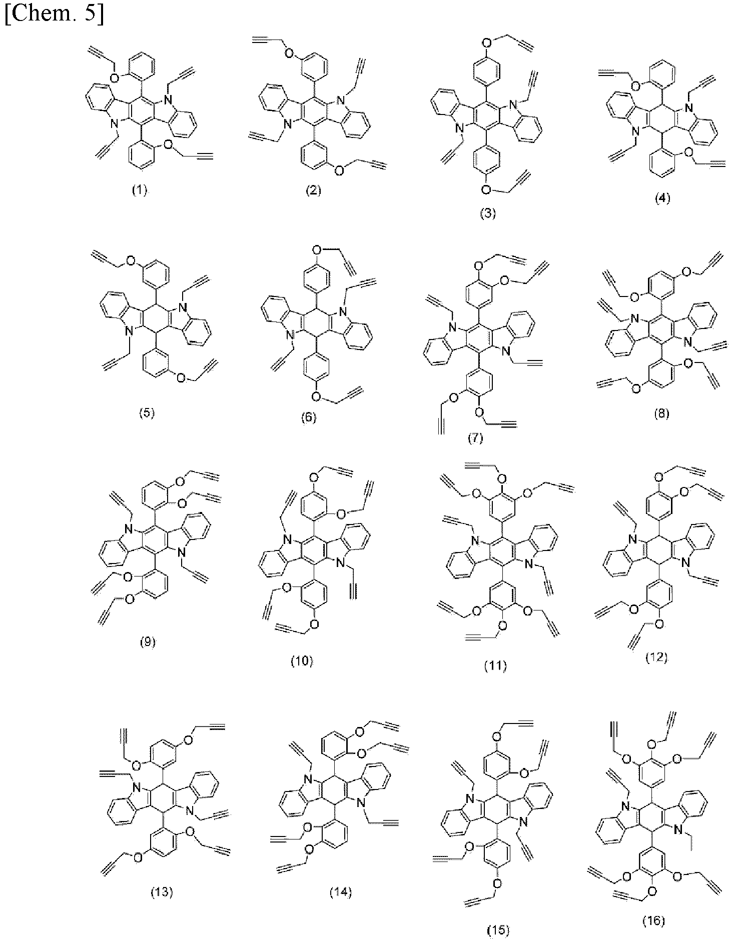

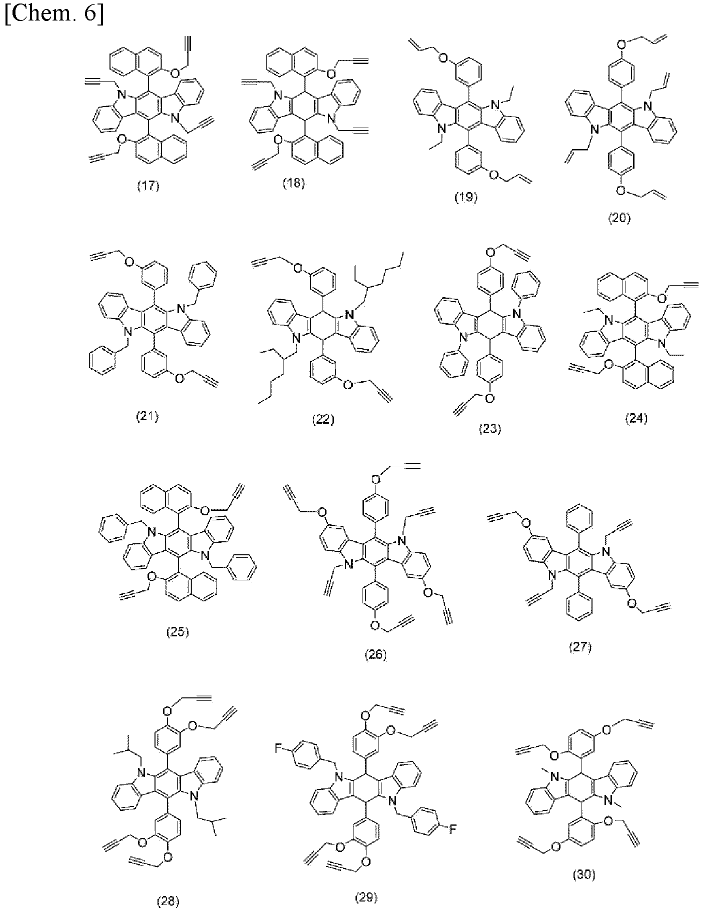

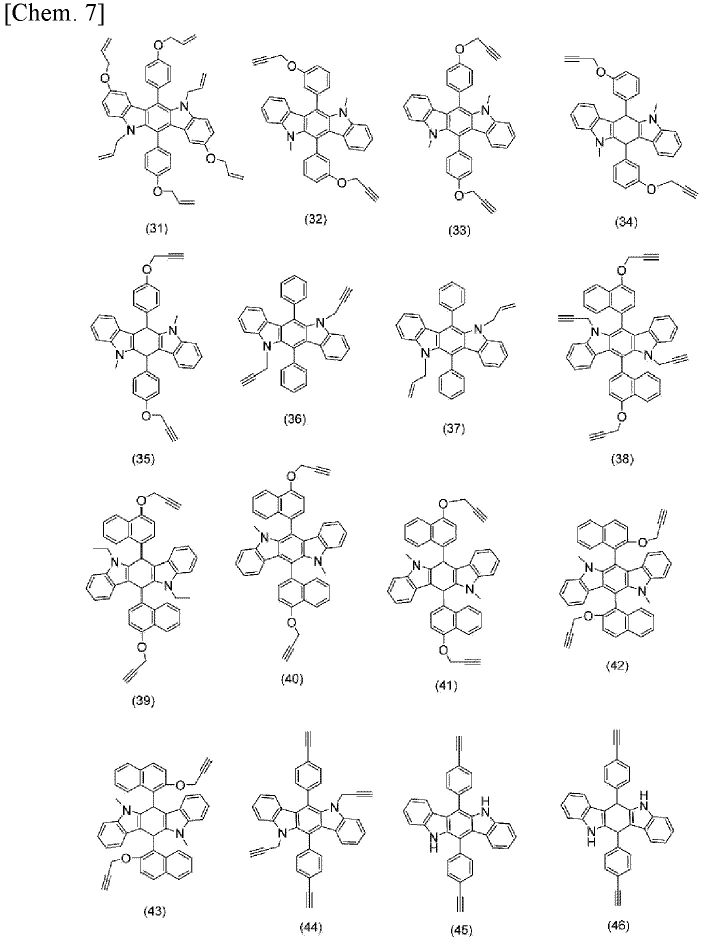

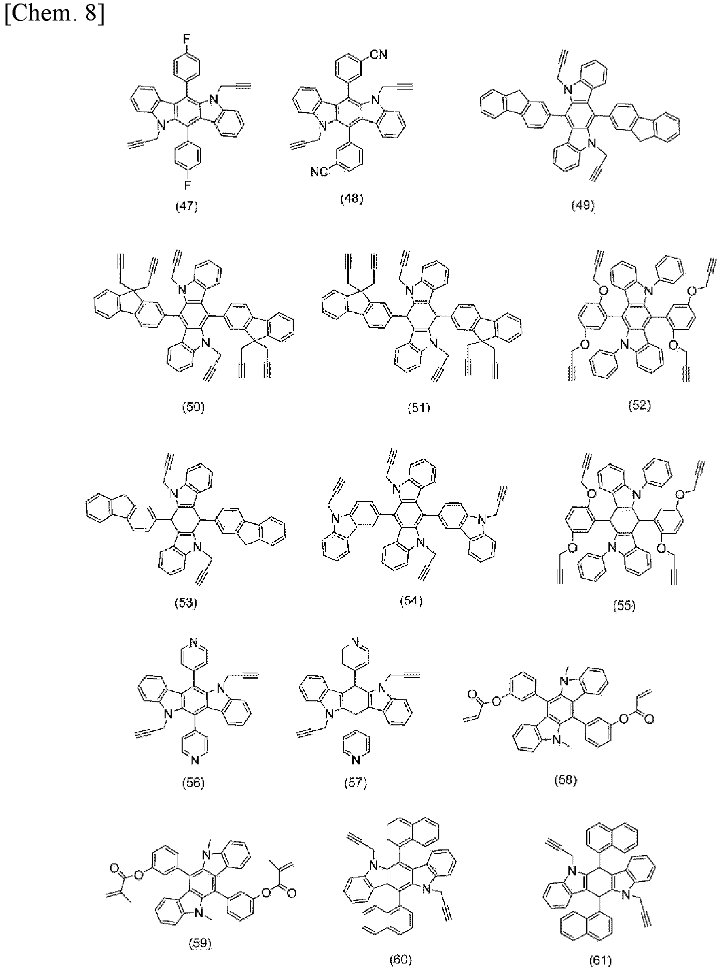

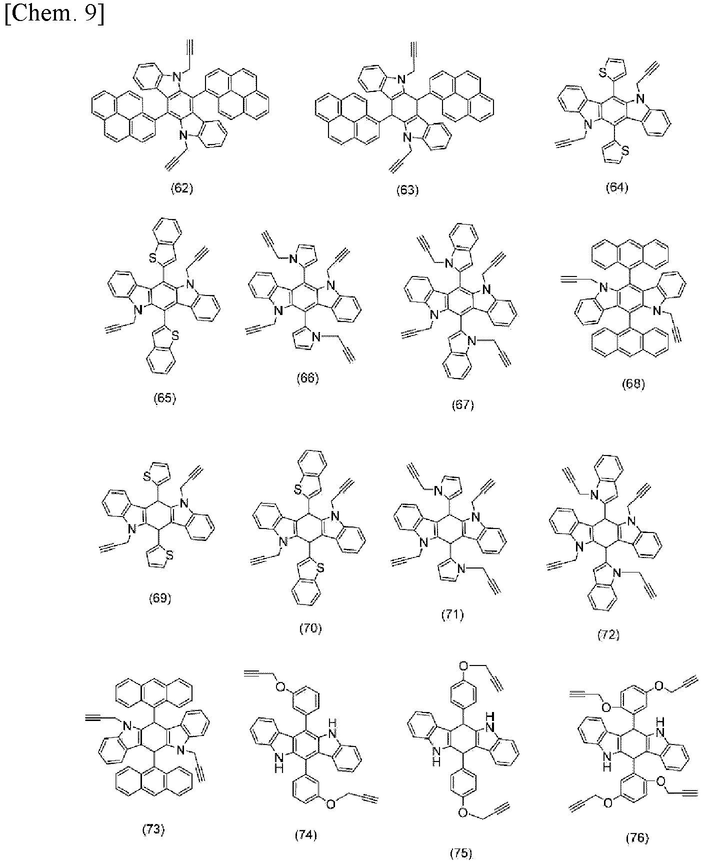

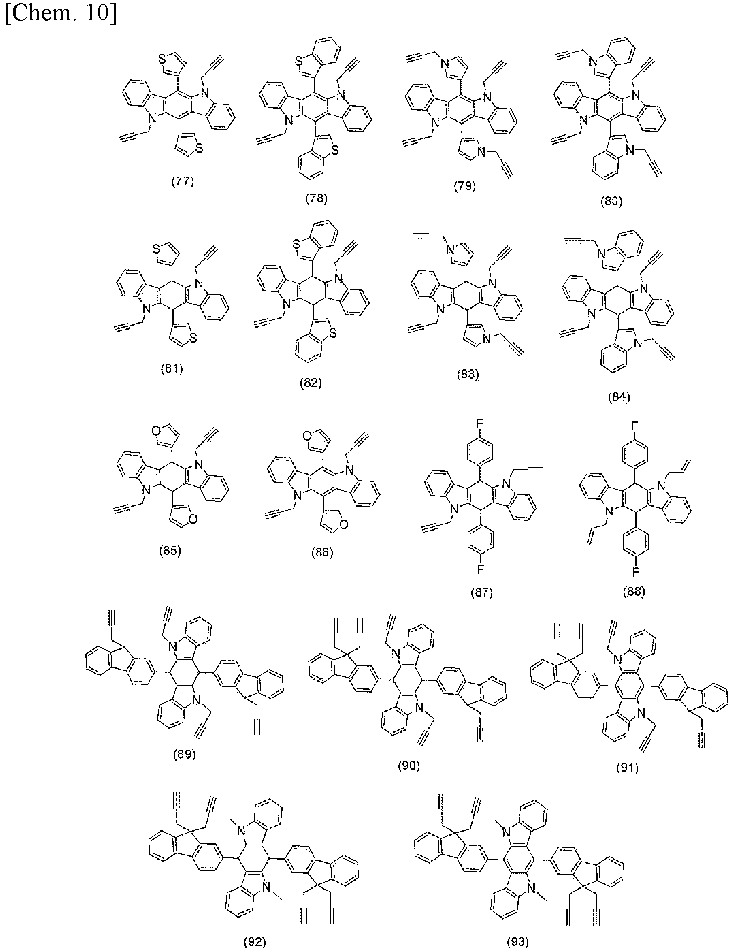

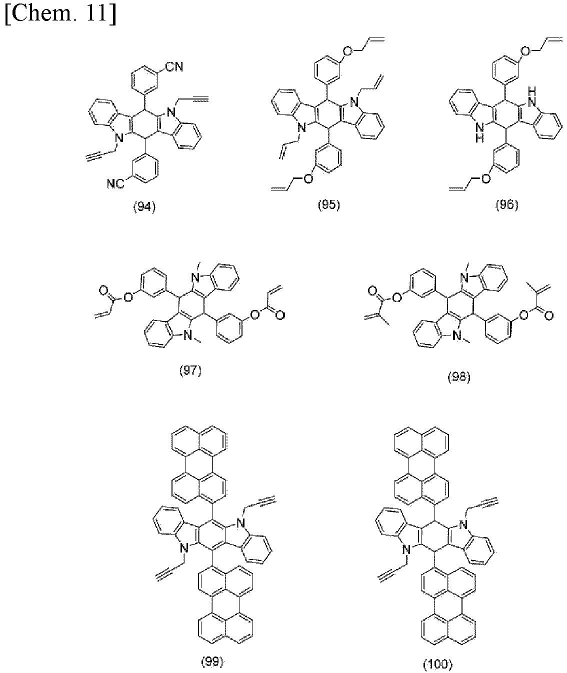

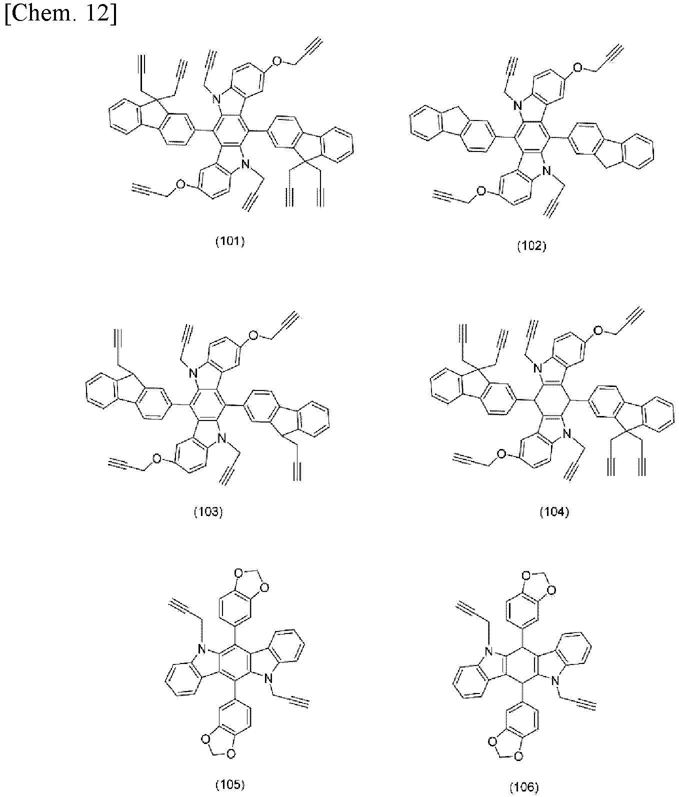

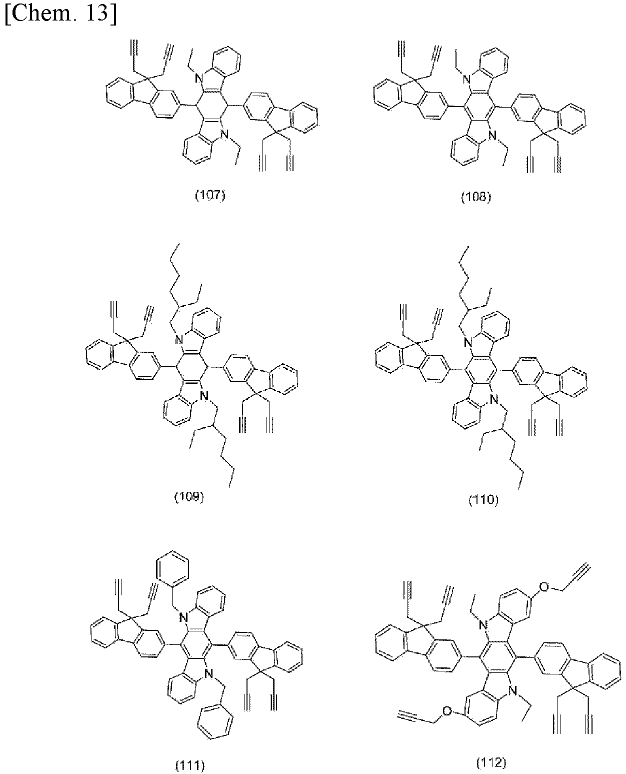

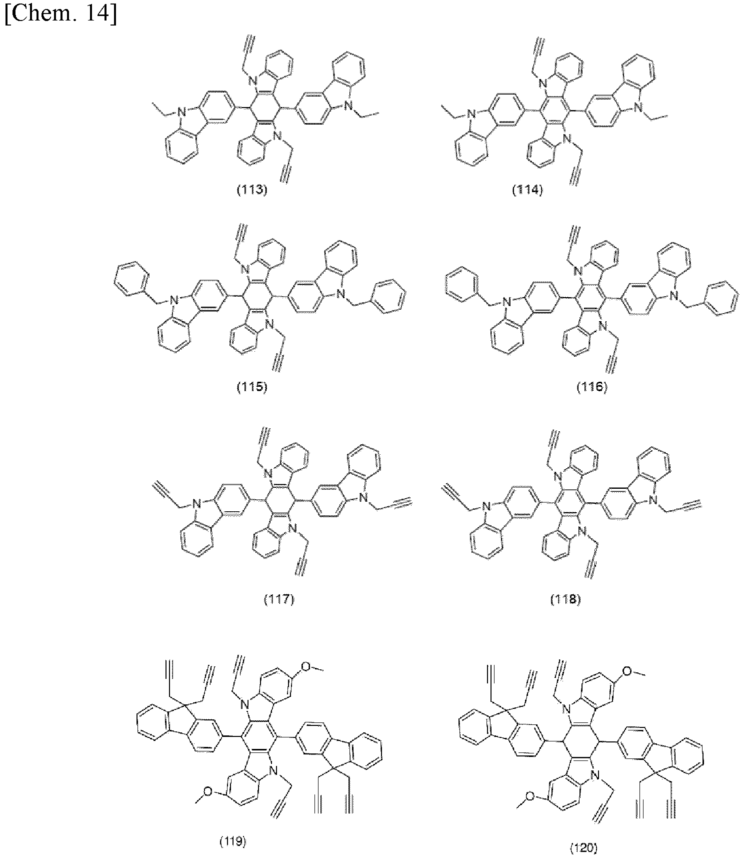

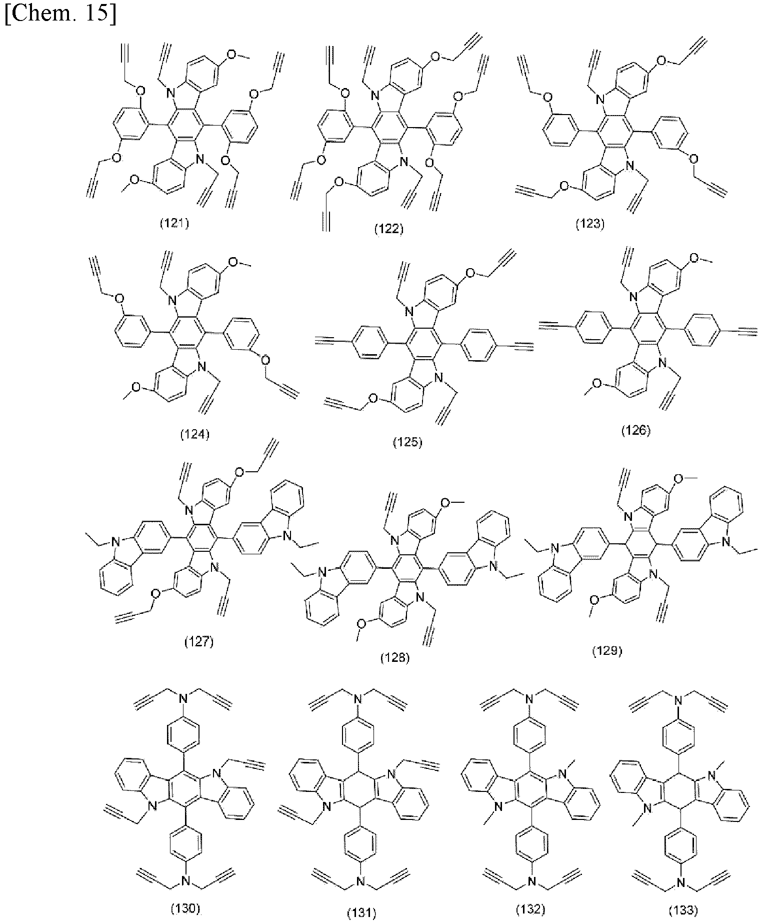

Specific examples of the compound (I) in which the reactive group is a phenolic hydroxy group include the following compounds (H1) to (H82).

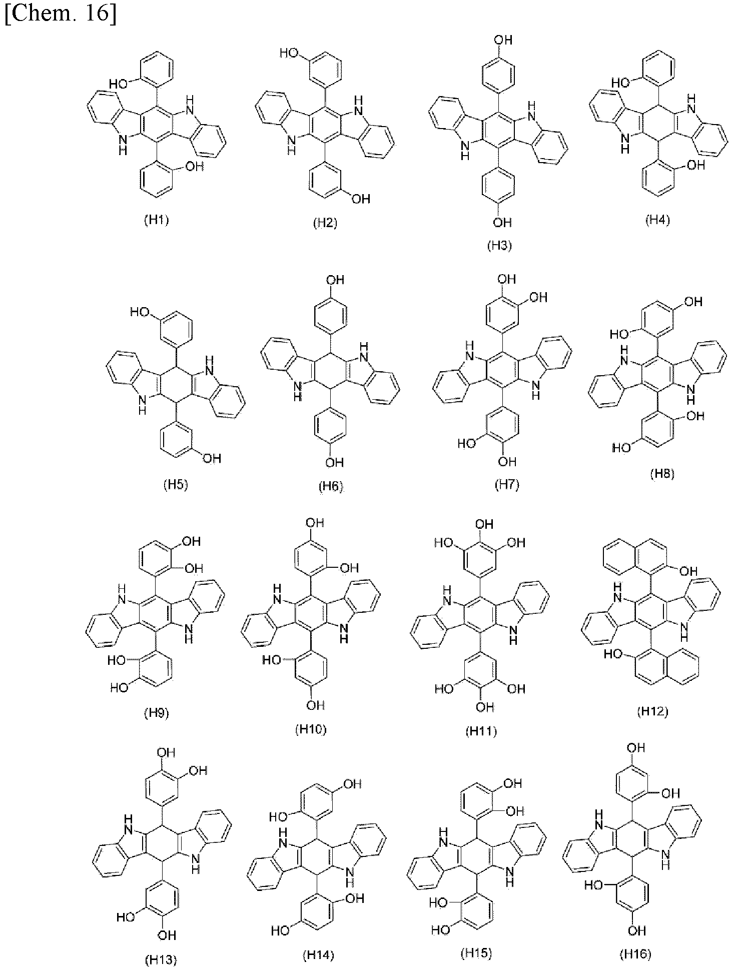

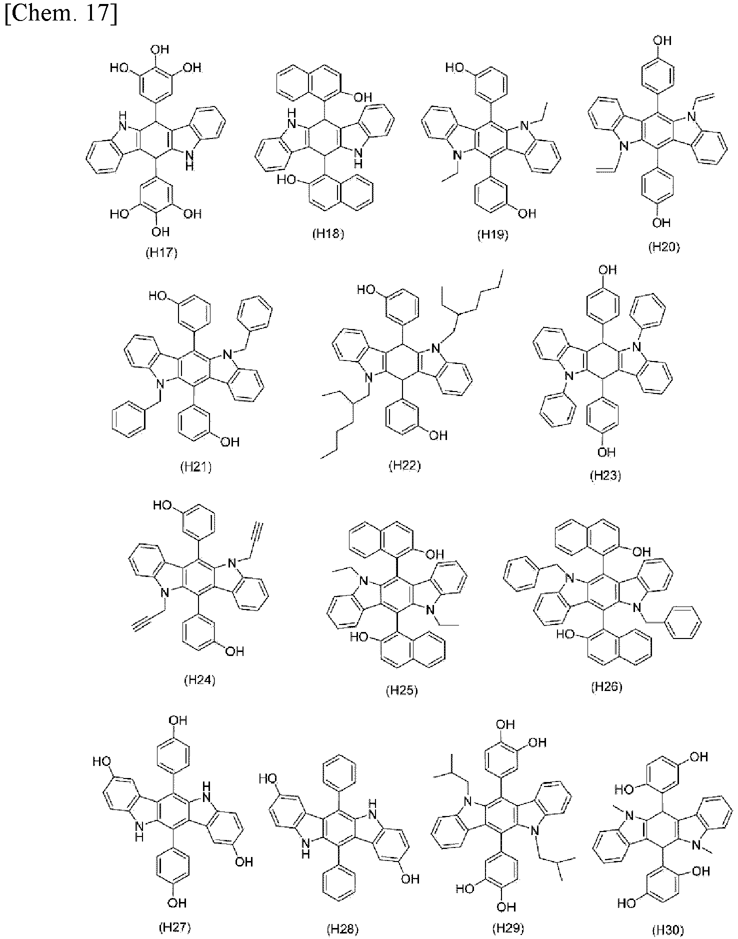

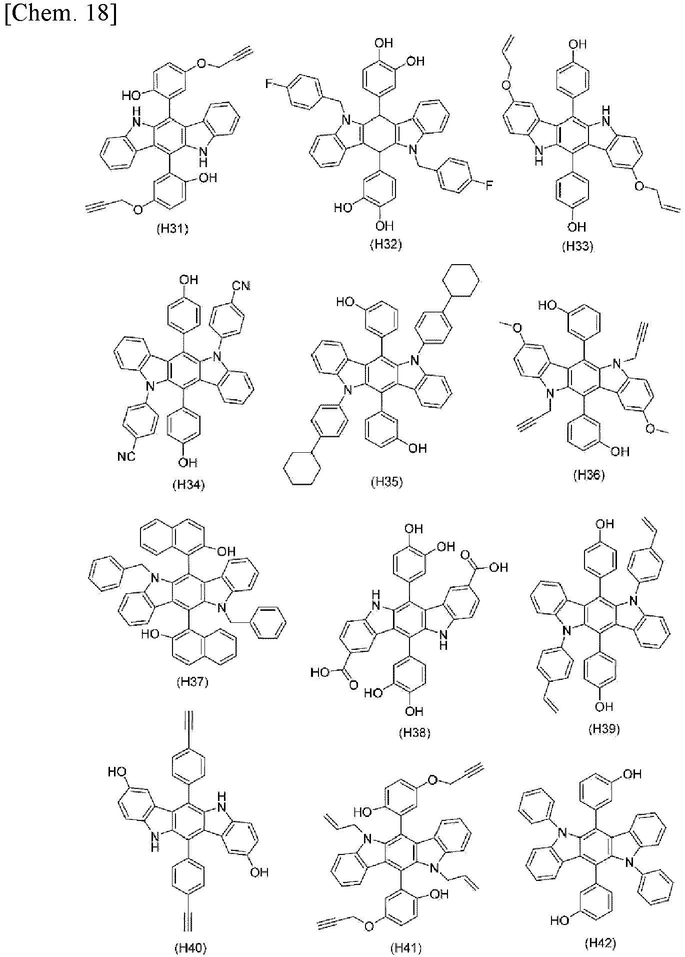

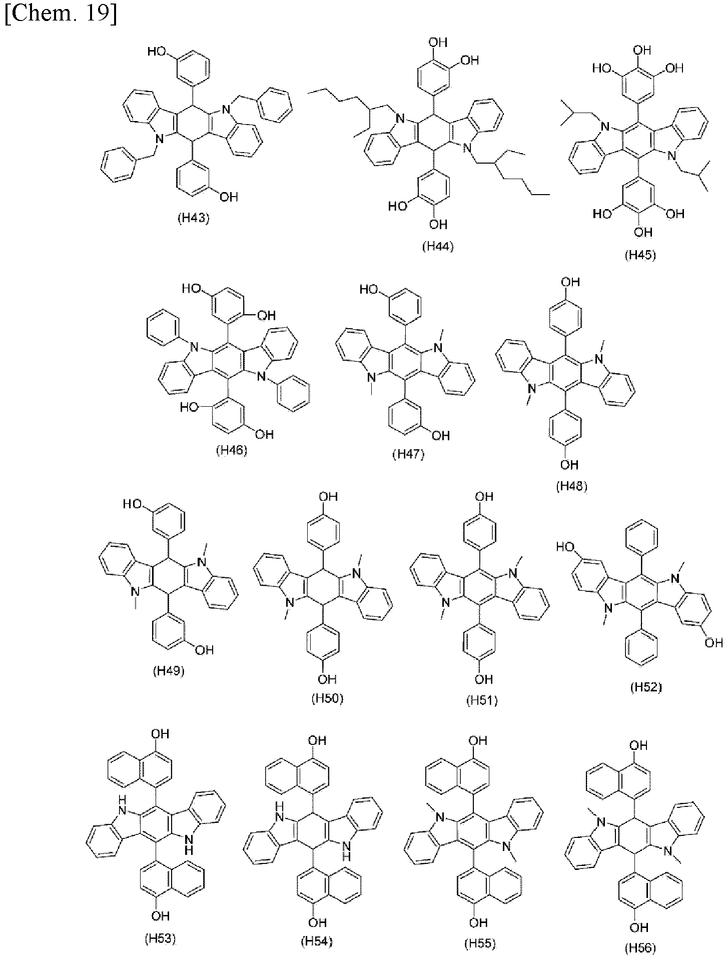

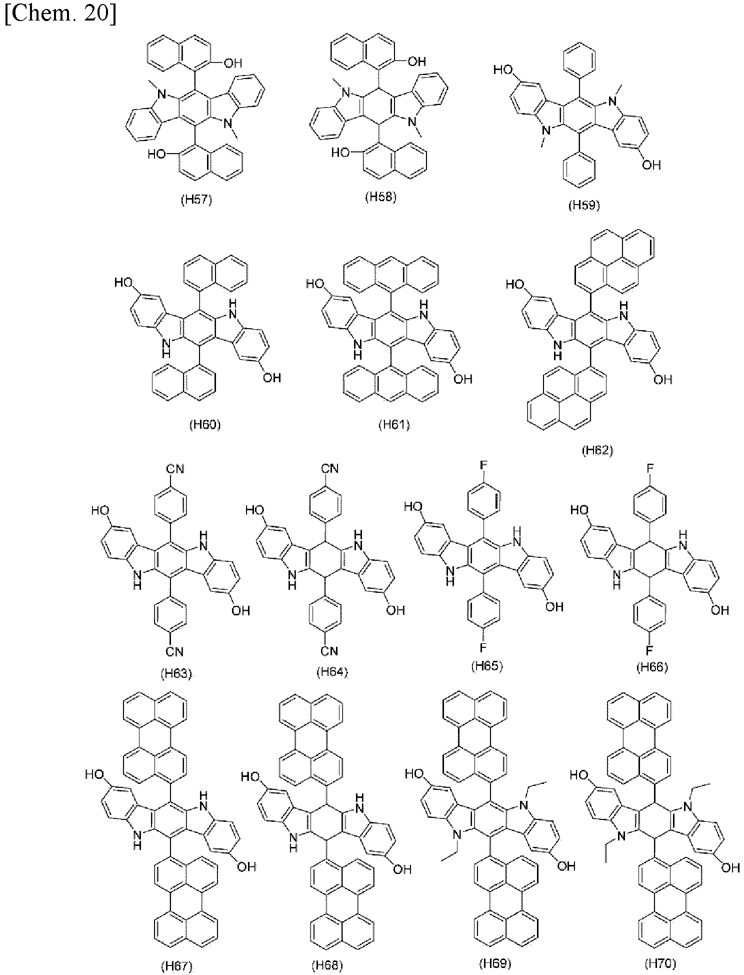

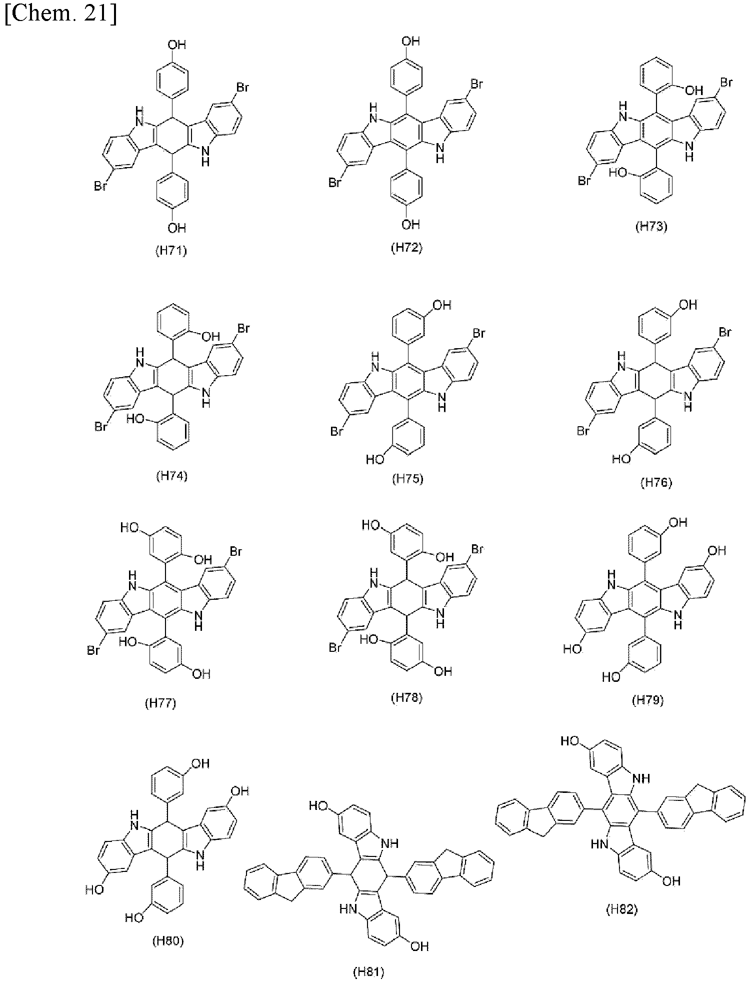

The compound (I) can be produced using a known method. Specifically, a tetrahydroindolocarbazole compound for a compound of the general formula (I) in which A is a cyclohexadiene ring can be produced by fusing indole with an aldehyde compound using an acid catalyst. Furthermore, a dihydroindolocarbazole compound for a compound of the general formula (I) in which A is a benzene ring can be produced by oxidizing the tetrahydroindolocarbazole compound with an oxidizing agent such as iodine or chloranil. Then, the compound (I) can be produced by introducing a reactive group into an amino group in the tetrahydroindolocarbazole compound or the dihydroindolocarbazole compound. For example, a compound of the general formula (I) in which X¹ and X² are phenyl groups, R⁵ and R¹⁰ are reactive groups R, and R¹, R², R³, R⁴, R⁶, R⁷, R⁸, and R⁹ are hydrogen atoms can be produced in the following manner.

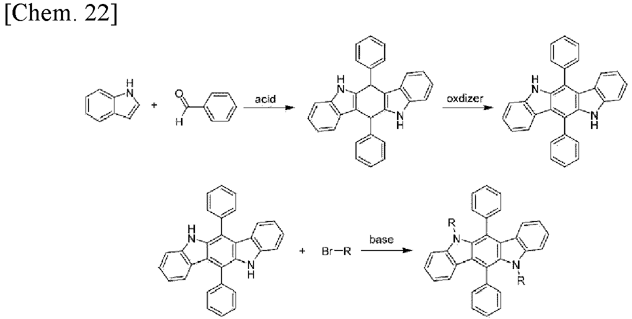

Also, a compound having a phenolic hydroxy group can be produced by using an aldehyde compound having a phenolic hydroxy group. For example, a compound of the general formula (I) in which X¹ and X² are 3-hydroxyphenyl groups, and R¹, R², R³, R⁴, R⁵, R⁶, R⁷, R⁸, R⁹, and R¹⁰ are hydrogen atoms can be produced in the following manner.

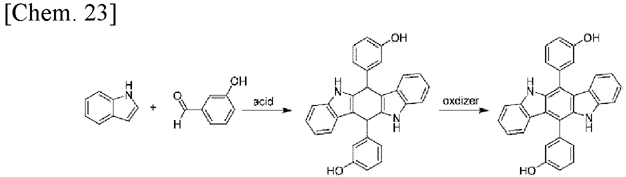

Three or more reactive groups R can be introduced into the molecule in the following manner.

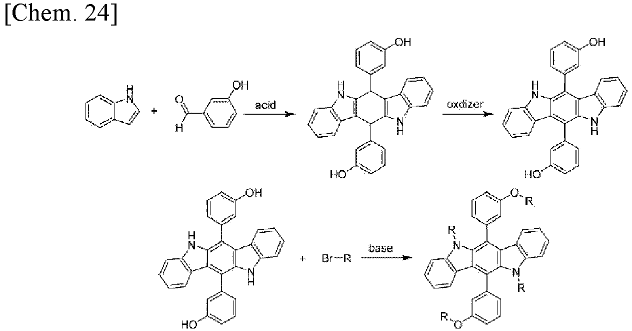

The composition of the present invention is characterized in that it contains the above-described compound (I). The composition, which contains the compound (I), can provide a cured product having excellent heat resistance.

The composition preferably contains the compound (I) in an amount of 20 to 100 mass% based on the solid content of the composition, in view of providing a cured product having excellent heat resistance. The content of the compound (I) is more preferably 40 to 100 mass%, and particularly preferably 60 to 100 mass%, based on the solid content. The term "solid content" here refers to the components of the composition, excluding a solvent, which will be described later.

The composition of the present invention may contain a cross-linking agent. A cross-linking agent means a compound that reacts with the compound (I) to link a plurality of the molecules together through chemical bonding to thereby be incorporated into a polymer resulting from the polymerization reaction, and thus modifies the physical and chemical properties of the polymer. When the composition contains a cross-linking agent, a cured product having even more excellent heat resistance can be obtained. Examples of the cross-linking agent include a phenolic compound, an epoxy compound, a cyanate compound, an amine compound, a benzoxazine compound, a melamine compound, a guanamine compound, a glycoluril compound, a urea compound, an isocyanate compound, and an azide compound.

Examples of the phenolic compound include: alkylphenols such as phenol, cresols, and xylenols; bisphenols such as bisphenol A, bisphenol F, bis(3-methyl-4-hydroxyphenyl)propane, and bis(4-hydroxyphenyl)-1-phenylethane; trisphenols such as α,α,α'-tris(4-hydroxyphenyl)-1-ethyl-4-isopropylbenzene; phenolic resins such as a phenolic novolac resin and a phenolaralkyl resin; and resinous phenolic derivatives such as linear trisphenols, methane-type trisphenols, linear tetrakisphenols, and radial hexanuclear compounds, which are represented by the general formulae below. 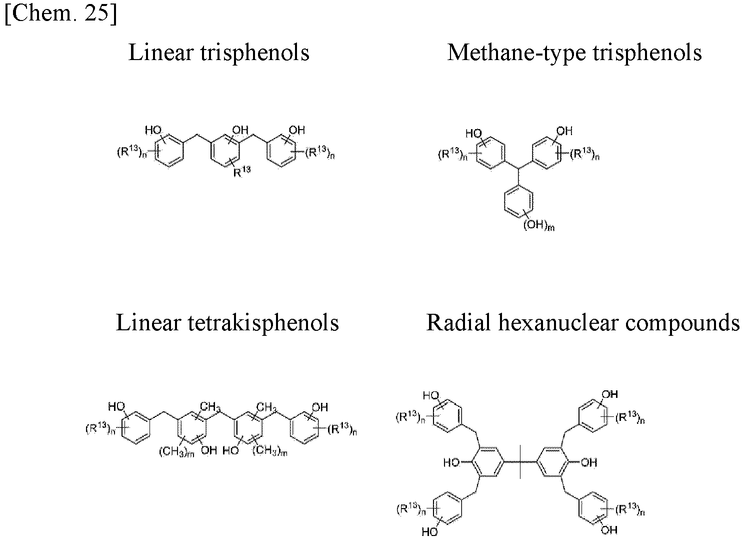 In the formulae, R¹³ represents an alkyl group having 1 to 4 carbon atoms, n represents an integer of 0 to 2, and m represents an integer of 0 to 1.

Examples of the epoxy compound include glycidyl ethers of the aforementioned phenolic compounds, tris(2,3-epoxypropyl)isocyanurate, trimethylolmethane triglycidyl ethers, and trimethylolpropane triglycidyl ethers.

Examples of the epoxy compound also include: polyglycidyl ether compounds of mononuclear polyvalent phenolic compounds such as hydroquinone, resorcin, pyrocatechol, and phloroglucinol; polyglycidyl ether compounds of polynuclear polyvalent phenolic compounds such as dihydroxynaphthalene, biphenol, methylenebisphenol (bisphenol F), methylenebis(orthocresol), ethylidenebisphenol, isopropylidenebisphenol (bisphenol A), 4,4'-dihydroxybenzophenone, isopropylidenebis(orthocresol), tetrabromobisphenol A, 1,3-bis(4-hydroxycumylbenzene), 1,4-bis(4-hydroxycumylbenzene), 1,1,3-tris(4-hydroxyphenyl)butane, 1,1,2,2-tetra(4-hydroxyphenyl)ethane, thiobisphenol, sulfobisphenol, oxybisphenol, phenol novolac, orthocresol novolac, ethyl phenol novolac, butyl phenol novolac, octyl phenol novolac, resorcinol novolac, and terpene phenol; polyglycidyl ethers of polyhydric alcohols such as ethylene glycol, propylene glycol, butylene glycol, hexanediol, polyglycol, thiodiglycol, glycerin, trimethylolpropane, pentaerythritol, sorbitol, and bisphenol A-ethylene oxide adducts; glycidyl esters of aliphatic, aromatic, or alicyclic polybasic acids such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, hexahydrophthalic acid, endomethylene tetrahydrophthalic acid, and the like, and homopolymers or copolymers of glycidyl methacrylate; epoxy compounds having glycidylamino groups, such as N,N-diglycidyl aniline, bis(4-(N-methyl-N-glycidylamino)phenyl)methane, and diglycidyl ortho-toluidine; epoxidized compounds of cyclic olefin compounds, such as vinylcyclohexene diepoxide, dicyclopentanediene diepoxide, 3,4-epoxy cyclohexylmethyl-3,4-epoxycyclohexane carboxylate, 3,4-epoxy-6-methylcyclohexylmethyl-6-methylcyclohexane carboxylate, and bis(3,4-epoxy-6-methylcyclohexylmethyl)adipate; epoxidized conjugated diene polymers such as epoxidized polybutadiene, epoxidized acrylonitrile-butadiene copolymers, and epoxidized styrene-butadiene copolymers; and heterocyclic compounds such as triglycidylisocyanurate.

Examples of the cyanate resin include a compound formed by replacing a hydroxy group in the above-described phenolic compound by a cyanate group.

Examples of the amine compound include: aromatic amines such as m-phenylenediamine, p-phenylenediamine, 4,4'-diaminodiphenylmethane, 4,4'-diaminodiphenylpropane, 4,4'-diaminodiphenyl ether, and 1,3-bis(4-aminophenoxy)benzene; alicyclic amines such as diaminocyclohexane, diaminodicyclohexylmethane, diaminodicyclohexylpropane, diaminobicyclo[2.2.1]heptane, and isophorone diamine; and aliphatic amines such as ethylenediamine, hexamethylenediamine, octamethylenediamine, decamethylenediamine, diethylenetriamine, and triethylenetetramine.

Examples of the benzoxazine compound include P-d type benzoxazine obtained from a diamine compound and a monofunctional phenolic compound, and F-a type benzoxazine obtained from an amine compound and a bifunctional phenolic compound.

Examples of the melamine compound include: hexamethylolmelamine; hexamethoxymethylmelamine; compounds obtained by methoxymethylating hexamethylolmelamine on one to six methylol groups thereof, or mixtures thereof; hexamethoxyethylmelamine; hexaacyloxymethylmelamine; and compounds obtained by acyloxymethylating hexamethylolmelamine on one to six methylol groups thereof, or mixtures thereof.

Examples of the guanamine compound include: tetramethylolguanamine; tetramethoxymethylguanamine; compounds obtained by methoxymethylating tetramethylolguanamine on one to four methylol groups thereof, or mixtures thereof; tetramethoxyethylguanamine, tetraacyloxyguanamine; and compounds obtained by acyloxymethylating tetramethylolguanamine on one to four methylol groups thereof, or mixtures thereof.

Examples of the glycoluril compound include: tetramethylolglycoluril; tetramethoxyglycoluril; tetramethoxymethylglycoluril; compounds obtained by methoxymethylating tetramethylolglycoluril on one to four methylol groups thereof, or mixtures thereof; and compounds obtained by acyloxymethylating tetramethylolglycoluril on one to four methylol groups thereof, or mixtures thereof.

Examples of the urea compound include: tetramethylolurea; tetramethoxymethylurea; and compounds obtained by methoxymethylating tetramethylolurea on one to four methylol groups thereof, or mixtures thereof; and tetramethoxyethylurea.

Examples of the isocyanate compound include: tolylene diisocyanate, diphenylmethane diisocyanate, hexamethylene diisocyanate, and cyclohexane diisocyanate. Examples of the azide compound include: 1,1'-biphenyl-4,4'-bisazide, and 4,4'-methylidenebisazide, 4,4'-oxybisazide.

As the cross-linking agent, a phenolic compound and a glycoluril compound are preferable, in view of obtaining a cured product having excellent heat resistance.

The content of the cross-linking agent is preferably 0.1 to 20 parts by mass, and more preferably 1 to 10 parts by mass, per 100 parts by mass of the compound (I). If the content of the cross-linking agent is less than the above-described range, the effect of improving the heat resistance may not be exhibited sufficiently.

The composition of the present invention may contain a solvent. The solvent means a compound that is liquid at 25°C and 1 atm, and may be any compound that is not classified as the above-described components (the compound represented by the general formula (I) and the cross-linking agent) or a polymerization initiator, which will be described later. Usually, a solvent in which the above-described components can be dissolved or dispersed as necessary can be used as the solvent. Examples include: ketones such as methyl ethyl ketone, methyl amyl ketone, diethyl ketone, acetone, methyl isopropyl ketone, methyl isobutyl ketone, cyclohexanone, and 2-heptanone; ether solvents such as ethyl ether, dioxane, tetrahydrofuran, 1,2-dimethoxyethane, 1,2-diethoxyethane, and dipropylene glycol dimethyl ether; ester solvents such as methyl acetate, ethyl acetate, n-propyl acetate, isopropyl acetate, n-butyl acetate, cyclohexyl acetate, ethyl lactate, dimethyl succinate, and Texanol; Cellosolve solvents such as ethylene glycol monomethyl ether and ethylene glycol monoethyl ether; alcoholic solvents such as methanol, ethanol, iso- or n-propanol, iso- or n-butanol, and amyl alcohol; ether ester solvents such as ethylene glycol monomethyl ether acetate, ethylene glycol monoethyl ether acetate, propylene glycol monomethyl ether acetate, dipropylene glycol monomethyl ether acetate, 3-methoxybutyl ether acetate, and ethoxyethyl ether propionate; BTX solvents such as benzene, toluene, and xylene; aliphatic hydrocarbon solvents such as hexane, heptane, octane, and cyclohexane; terpene hydrocarbon oils such as turpentine, D-limonene, and pinene; mineral spirits, Swasol #310 (Cosmo Matsuyama Oil Co., Ltd.), Solvesso #100 (Exxon Chemical Company), and other paraffin solvents; halogenated aliphatic hydrocarbon solvents such as carbon tetrachloride, chloroform, trichloroethylene, methylene chloride, and 1,2-dichloroethane; halogenated aromatic hydrocarbon solvents such as chlorobenzene; carbitol solvents; aniline; triethylamine; pyridine; acetic acid; acetonitrile; carbon disulfide; N,N-dimethylformamide; N,N-dimethylacetamide (DMAc); N-methylpyrrolidone; dimethyl sulfoxide; and water. One of these solvents may be used alone, or two or more thereof may be used as a mixed solvent.

Of these solvents, ketones and ether ester solvents, in particular, propylene glycol monomethyl ether acetate and cyclohexanone are preferable in view of their great ability to dissolve the compound (I) and the cross-linking agent.

The content of the solvent is preferably from 50 to 99 parts by mass, and more preferably from 70 to 95 parts by mass, per 100 parts by mass of the composition.

The composition of the present invention may contain a polymerization initiator. The polymerization initiator means a compound that can generate active species upon, for example, heating or irradiation with active energy rays to thereby initiate polymerization, and known polymerization initiators can be used. Examples of the polymerization initiator include acid generators and radical polymerization initiators. When the composition contains a polymerization initiator, increase in the polymerization reaction rate and combination use of multiple polymerization reactions can be expected, and it can be thus expected that curing of the composition is accelerated.

Polymerization initiators can be classified into photoinitiators, which generate active species upon irradiation with active energy rays, and thermal initiators, which generate active species upon heating. Thermal initiators are preferred because of their high reactivity. Examples of the active species to be generated include acids, bases, and radicals. Acids are preferred because of their high reactivity with phenolic hydroxy groups. Examples of the photoinitiators include photo-radical polymerization initiators and photo acid generators. Examples of the thermal initiators include thermal radical polymerization initiators and thermal acid generators. When the reactive group is a phenolic hydroxy group, the polymerization initiator is preferably a thermal acid generator.

The composition of the present invention may contain an acid generator as the polymerization initiator in order to accelerate the curing reaction of the cross-linking agent. The acid generator may be any compound that can generate an acid under predetermined conditions, and examples thereof include onium salts such as sulfonium salts, iodonium salts, and ammonium salts.

Specific examples of the acid generator include onium salts such as tetramethylammonium trifluoromethanesulfonate, tetramethylammonium nonafluorobutanesulfonate, triethylammonium nonafluorobutanesulfonate, pyridinium nonafluorobutanesulfonate, triethylammonium camphorsulfonate, pyridinium camphorsulfonate, tetra-n-butylammonium nonafluorobutanesulfonate, tetraphenylammonium nonafluorobutanesulfonate, tetramethylammonium p-toluenesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, bis(p-tert-butoxyphenyl)phenylsulfonium trifluoromethanesulfonate, tris(p-tert-butoxyphenyl)sulfonium trifluoromethanesulfonate, triphenylsulfonium p-toluenesulfonate, (p-tert-butoxyphenyl)diphenylsulfonium p-toluenesulfonate, bis(p-tert-butoxyphenyl)phenylsulfonium p-toluenesulfonate, tris(p-tert-butoxyphenyl)sulfonium p-toluenesulfonate, triphenylsulfonium nonafluorobutanesulfonate, triphenylsulfonium butanesulfonate, trimethylsulfonium trifluoromethanesulfonate, trimethylsulfonium p-toluenesulfonate, cyclohexylmethyl(2-oxocyclohexyl)sulfonium trifluoromethanesulfonate, cyclohexylmethyl(2-oxocyclohexyl)sulfonium p-toluenesulfonate, dimethylphenylsulfonium trifluoromethanesulfonate, dimethylphenylsulfonium p-toluenesulfonate, dicyclohexylphenylsulfonium trifluoromethanesulfonate, dicyclohexylphenylsulfonium p-toluenesulfonate, trinaphthylsulfonium trifluoromethanesulfonate, cyclohexylmethyl(2-oxocyclohexyl)sulfonium trifluoromethanesulfonate, (2-norbomyl)methyl(2-oxocyclohexyl)sulfonium trifluoromethanesulfonate, ethylenebis[methyl(2-oxocyclopentyl)sulfonium trifluoromethanesulfonate], and 1,2'-naphthylcarbonylmethyltetrahydrothiophenium triflate.

A thermal acid generator, which generates an acid upon exposure to heat, is particularly preferable in view of providing good curing properties.

The content of the acid generator is preferably 50 to 150 parts by mass, and more preferably 80 to 120 parts by mass, per 100 parts by mass of the cross-linking agent. When the content of the acid generator is within the above-described range, a composition having excellent curing properties can be obtained.

When the compound (I) has a carbon-carbon double bond, the composition of the present invention may contain a radical polymerization initiator as the polymerization initiator. Any of photo-radical polymerization initiators and thermal radical polymerization initiators can be used as the radical polymerization initiator, as the radical polymerization initiator.

Examples of the photo-radical polymerization initiators include: benzoins such as benzoin, benzoin methyl ether, benzoin propyl ether, and benzoin butyl ether; benzyl ketals such as benzyl dimethyl ketal; acetophenones such as acetophenone, 2,2-dimethoxy-2-phenylacetophenone, 2,2-diethoxy-2-phenylacetophenone, 1-benzyl-1-dimethylamino-1-(4'-morpholinobenzoyl)propane, 2-morpholyl-2-(4'-methylmercapto)benzoylpropane, 2-methyl-1-[4-(methylthio)phenyl]-2-morpholino-propan-1-one, 1-hydroxycyclohexylphenyl ketone, 1-hydroxy-1-benzoylcyclohexane, 2-hydroxy-2-benzoylpropane, 2-hydroxy-2-(4'-isopropyl)benzoylpropane, N,N-dimethylaminoacetophenone, 1,1-dichloroacetophenone, 4-butylbenzoyltrichloromethane, and 4-phenoxybenzoyldichloromethane; anthraquinones such as 2-methylanthraquinone, 1-chloroanthraquinone, and 2-amylanthraquinone; thioxanthones such as 2,4-dimethylthioxanthone, 2,4-diethylthioxanthone, 2-chlorothioxanthone, and 2,4-diisopropylthioxanthone; ketals such as acetophenone dimethyl ketal and benzyl dimethyl ketal; benzophenones such as benzophenone, methylbenzophenone, 4,4'-dichlorobenzophenone, 4,4'-bisdiethylaminobenzophenone, Michler's ketone, and 4-benzoyl-4'-methyldiphenyl sulfide; oxides such as 2,4,6-trimethylbenzoyldiphenylphosphine oxide and bis(2,4,6-trimethylbenzoyl)-phenylphosphine oxide; carbazoles such as 3-(2-methyl-2-morpholinopropionyl)-9-methylcarbazole; α-dicarbonyls such as benzyl and methyl benzoylformate; oxime esters such as compounds disclosed in JP 2000-80068A, JP 2001-233842A, JP 2005-97141A, JP 2006-516246T, Japanese Patent No. 3860170, Japanese Patent No. 3798008, WO 2006/018973, JP 2011-132215A, and WO 2015/152153; triazines such as p-methoxyphenyl-2,4-bis(trichloromethyl)-s-triazine, 2-methyl-4,6-bis(trichloromethyl)-s-triazine, 2-phenyl-4,6-bis(trichloromethyl)-s-triazine, 2-naphthyl-4,6-bis(trichloromethyl)-s-triazine, and 2-(p-butoxystyryl)-s-triazine; and benzoyl peroxide, 2,2'-azobisisobutyronitrile, ethyl anthraquinone, 1,7-bis(9'-acridinyl)heptane, thioxanthone, 1-chlor-4-propoxythioxanthone, isopropylthioxanthone, diethylthioxanthone, benzophenone, phenyl biphenyl ketone, 4-benzoyl-4'-methyldiphenyl sulfide, 2-(p-butoxystyryl)-5-trichloromethyl-1,3,4-oxadiazole, 9-phenylacridine, 9,10-dimethyl benzphenazine, benzophenone/Michler's ketone, hexaarylbiimidazole/mercaptobenzimidazole, and thioxanthone/amine.

Examples of the thermal radical polymerization initiators include: peroxides such as benzoyl peroxide, 2,4-dichlorobenzoyl peroxide, 1,1-di(t-butylperoxy)-3,3,5-trimethylcyclohexane, 4,4-di(t-butylperoxy)butyl valerate, and dicumyl peroxide; azo compounds such as 2,2'-azobisisobutyronitrile; and tetramethylthiraum disulfide.

The content of the radical polymerization initiator is preferably from 0.1 to 20 parts by mass, more preferably from 0.5 to 15 parts by mass, and even more preferably from 1 to 10 parts by mass, per 100 parts by mass of the compound (I) having a carbon-carbon double bond, in view of obtaining a composition having excellent curing properties and providing a cured product excellent in heat resistance and solvent resistance.

In addition to the above-described various components, the composition of the present invention may contain other components as necessary.

Examples of the other components include: various additives such as an inorganic filler, an organic filler, a silane coupling agent, a colorant, a photosensitizer, an antifoaming agent, a thickener, a thixotropic agent, a surfactant, a leveling agent, a flame retardant, a plasticizer, a stabilizer, a polymerization inhibitor, an ultraviolet absorber, an antioxidant, an antistatic agent, a flow modifier, and an adhesion promoter.

The composition of the present invention can be cured by heating, for example, on a heating plate such as a hot plate, or in an atmospheric oven, an inert gas oven, a vacuum oven, or a hot air circulating oven.

The heating temperature during thermal curing can be selected as appropriate according to the type of the reactive group. For example, when the reactive group is a carbon-carbon triple bond or a phenolic hydroxy group, the heating temperature is preferably 200 to 400°C, and more preferably 250 to 350°C. The curing time is not particularly limited, and is preferably 1 to 60 minutes, and more preferably 1 to 30 minutes, in view of improving the productivity.

The compound and the composition of the present invention have excellent heat resistance, and can therefore be suitably used in applications to electronic component, such as semiconductor encapsulants, circuit boards, build-up films, build-up PCBs, semiconductor resists, semiconductor hard masks, and multilayer flexible films. Coating materials, adhesives, heat-resistant members, and electronic components in which the composition of the present invention is used can be suitably used in various applications, and examples of the applications include, but are not limited to, industrial machine parts, general machine parts, parts for automobiles, railroads, vehicles, and the like, aerospace-related parts, electronic and electrical parts, building materials, container and packaging members, household goods, sports and leisure goods, and casing members for wind power generation.

The composition of the present invention can be easily embedded into uneven substrates, and accordingly, the composition can also be used as a gap fill material to be filled into recesses formed in substrates (film embedded for planarization), and can be used to form an insulating material to be filled into recesses formed in substrates (film embedded for insulation), a barrier material, a resist material for semiconductor, and an insulating film for through-silicon vias, for example.

Furthermore, a compound of the present invention that has a phenolic hydroxy group can be used as a starting material for polymers such as polyesters, polycarbonates, and phenolic resins.

### Examples

Hereinafter, the present invention will be described more specifically by way of examples and comparative examples, but the present invention is not limited by the examples below.

### Synthesis of Compound (P-1)

40.0 g (341 mmol) of indole, 47.2 g (341 mmol) of 2,5-dihydroxybenzaldehyde, and 243 g of acetonitrile were placed in a reaction flask equipped with a reflux tube and stirred until completely dissolved. After water-cooling, 5.7 g (34 mmol) of 48% hydrobromic acid was slowly added dropwise. After it was confirmed that heat generation had subsided, the mixture was returned to room temperature and then stirred for 2 hours. The reaction solution was cooled to 10°C, and the precipitate was collected by filtering and washed with 100 g of acetonitrile. The filter cake was dried under reduced pressure at 40°C to obtain compound (P-1) as a gray powder.
Actual yield: 33.2 g (percent yield: 41%)
¹H-NMR (DMSO-d₆) δ/ppm: 6.00 (s, 2H), 6.04-7.33 (m, 14H), 8.27 (s, 2H), 9.25 (s, 2H), 10.42 (s, 2H)

### Synthesis of Compound (P-2)

Then, 30.0 g (63.2 mmol) of compound (P-1), 19.3 g (75.9 mmol) of iodine, and 90 g of acetonitrile were placed in a reaction flask equipped with a reflux tube, the temperature was raised, and a reaction was carried out at 80°C for 14 hours. The reaction solution was cooled to room temperature, 30 g of a 10% aqueous sodium thiosulfate solution was added dropwise, and the mixture was cooled to 10°C under stirring. The precipitate was collected by filtering and washed with 50 g of acetonitrile, and the filter cake was dried under reduced pressure at 40°C to obtain compound (P-2) as a pale brown powder.
Actual yield: 29.7 g (percent yield: 99%)
¹H-NMR (DMSO-d₆) δ/ppm: 6.80-7.43 (m, 14H), 8.60 (s, 2H), 8.89 (s, 2H), 10.27 (s, 2H)

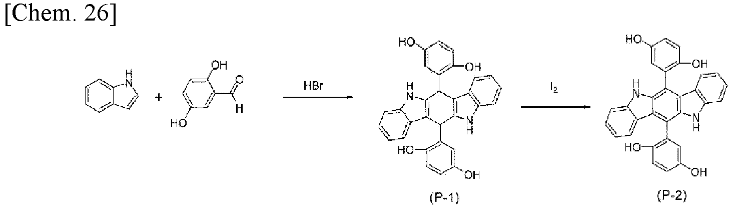

### Synthesis of Compound (P-3)

Compound (P-3) was obtained as a gray powder in the same manner as for the synthesis of compound (P-1), except that 3,4-dihydroxybenzaldehyde was used instead of 2,5-dihydroxybenzaldehyde.
Actual yield: 50.2 g (percent yield: 62%)
¹H-NMR (DMSO-d₆) δ/ppm: 5.44 (s, 2H), 6.49-7.26 (m, 14H), 8.63 (s, 4H), 10.55 (s, 2H)

### Synthesis of Compound (P-4)

Compound (P-4) was obtained as a gray powder in the same manner as for the synthesis of compound (P-2), except that compound (P-3) was used instead of compound (P-1).
Actual yield: 27.6 g (percent yield: 92%)
¹H-NMR (DMSO-d₆) δ/ppm: 6.85-7.43 (m, 14H), 9.10 (s, 2H), 9.22 (s, 2H), 10.39 (s, 2H)

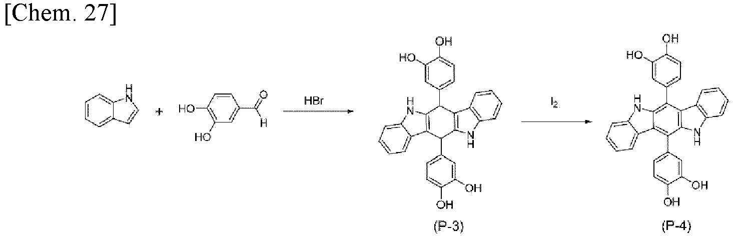

### Synthesis of Compound (P-5)

29.3 g (250 mmol) of indole, 30.5 g (250 mmol) of 4-hydroxybenzaldehyde, and 166 g of acetonitrile were placed in a reaction flask equipped with a reflux tube and stirred until completely dissolved. After water-cooling, 4.2 g (25 mmol) of 48% hydrobromic acid was slowly added dropwise. After it was confirmed that heat generation had subsided, the mixture was returned to room temperature and then stirred for 2 hours. The reaction solution was cooled to 10°C, and the precipitate was collected by filtering and washed with 70 g of acetonitrile. The filter cake was dried under reduced pressure at 40°C to obtain compound (P-5) as a gray powder.
Actual yield: 34.3 g (percent yield: 65%)
¹H-NMR (DMSO-d₆) δ/ppm: 5.52 (s, 2H), 6.64-7.23 (m, 16H), 9.17 (s, 2H), 10.56 (s, 2H)

### Synthesis of Compound (P-6)

Then, 26.6 g (60 mmol) of compound (P-5), 18.3 g (72 mmol) of iodine, and 80 g of acetonitrile were placed in a reaction flask equipped with a reflux tube, the temperature was raised, and a reaction was carried out at 80°C for 14 hours. The reaction solution was cooled to room temperature, 25 g of a 10% aqueous sodium thiosulfate solution was added dropwise, and the mixture was cooled to 10°C under stirring. The precipitate was collected by filtering and washed with 50 g of acetonitrile, and the filter cake was dried under reduced pressure at 40°C to obtain compound (P-6) as a pale yellow powder.
Actual yield: 24.3 g (percent yield: 92%)
¹H-NMR (DMSO-d₆) δ/ppm: 6.81-7.47 (m, 16H), 9.67 (s, 2H), 10.40 (s, 2H)

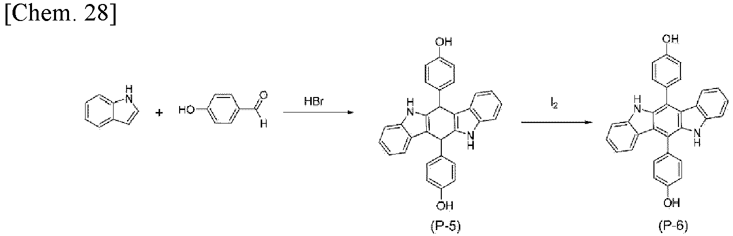

### Synthesis of Compound (P-7)

Compound (P-7) was obtained as a pale yellow powder in the same manner as for the synthesis of compound (P-5), except that 3-hydroxybenzaldehyde was used instead of 4-hydroxybenzaldehyde.
Actual yield: 28.5 g (percent yield: 54%)
¹H-NMR (DMSO-d₆) δ/ppm: 5.57 (s, 2H), 6.59-7.25 (m, 16H), 9.16 (s, 2H), 10.66 (s, 2H)

### Synthesis of Compound (P-8)

Compound (P-7) was obtained as a pale yellow powder in the same manner as for the synthesis of compound (P-6), except that compound (P-7) was used instead of compound (P-5).
Actual yield: 20.6 g (percent yield: 78%)
¹H-NMR (DMSO-d₆) δ/ppm: 6.83-7.49 (m, 16H), 9.68 (s, 2H), 10.49 (s, 2H)

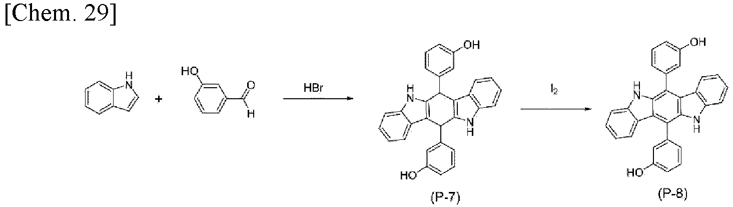

### Synthesis of Compound (P-9)

39.4 g (300 mmol) of 1-methylindole, 51.7 g (300 mmol) of 2-hydroxy-1-naphthaldehyde, and 250 g of acetonitrile were placed in a reaction flask equipped with a reflux tube and stirred until completely dissolved. After water-cooling, 5.1 g (30 mmol) of 48% hydrobromic acid was slowly added dropwise. After it was confirmed that heat generation had subsided, the mixture was returned to room temperature and then stirred for 2 hours. The precipitate of the reaction solution was collected by filtering and washed with 100 g of acetonitrile, and the filter cake was dried under reduced pressure at 40°C to obtain (P-9) as a pinkish gray powder.
Actual yield: 63.0 g (percent yield: 73.3%)
¹H-NMR (DMSO-d₆) δ/ppm: 3.67 (s, 6H), 6.84 (s, 2H), 7.06-8.34 (m, 20H), 9.89 (s, 2H)

### Synthesis of Compound (P-10)

Then, 22.8 g (40.0 mmol) of compound (P-9), 10.8 g (44.0 mmol) of chloranil, and 350 g of o-xylene were placed in a reaction flask equipped with a reflux tube, the temperature was raised, and a reaction was carried out at 140°C for 7 hours. The reaction solution was cooled to room temperature, and the solvent was removed. Then, 200 g of hexane and 100 g of isopropanol were added to the residue, and the mixture was stirred at room temperature for 30 minutes. The precipitate was collected by filtering and washed with 50 g of acetonitrile, and the filter cake was dried under reduced pressure at 40°C to obtain compound (P-10) as a reddish brown powder.
Actual yield: 7.0 g (percent yield: 30.8%)
¹H-NMR (DMSO-d₆) δ/ppm: 3.84 (s, 6H), 7.08-8.25 (m, 20H), 9.67 (s, 2H)

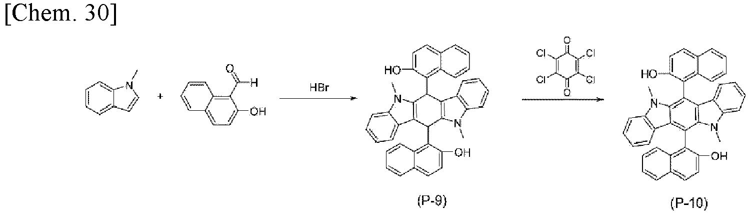

### Synthesis of Compound (13)

5.21 g (11 mmol) of compound (P-1) and 50 g of dimethyl sulfoxide (DMSO) were placed in a reaction flask equipped with a reflux tube, and 5.50 g (66 mmol) of a 48% aqueous sodium hydroxide solution was added dropwise while stirring at room temperature under a nitrogen stream. Subsequently, 7.50 g (63 mmol) of 3-bromo-1-propyne was added dropwise, and the mixture was then stirred at room temperature for 2 hours. The reaction solution was poured into 300 g of water, and after stirring for 30 minutes, the precipitate was collected by filtering. 50 g of isopropanol was added to the collected filter cake, followed by stirring for 30 minutes. Then, the mixture was filtered, and the residue was dried under reduced pressure at 40°C to obtain compound (13) as a pale yellow powder.
Actual yield: 4.0 g (percent yield: 57%)
¹H-NMR (DMSO-d₆) δ/ppm: 2.98 (t, 2H), 3.24 (t, 2H), 3.75 (t, 2H), 4.62-4.88 (m, 12H), 6.11 (s, 2H), 6.80-7.69 (m, 14H)

### Synthesis of Compound (8)

5.20 g (11 mmol) of compound (P-2) and 50 g of dimethyl sulfoxide (DMSO) were placed in a reaction flask equipped with a reflux tube, and 5.50 g (66 mmol) of a 48% aqueous sodium hydroxide solution was added dropwise while stirring at room temperature under a nitrogen stream. Subsequently, 7.50 g (63 mmol) of 3-bromo-1-propyne was added dropwise, and the mixture was then stirred at room temperature for 2 hours. The reaction solution was poured into 300 g of water, and after stirring for 30 minutes, the precipitate was collected by filtering. The collected filter cake was dissolved in 50 g of ethyl acetate, and then, 50 g of water was added. The mixture was stirred for 30 minutes, followed by oil/water separation. The solvent was removed from the organic layer, and the residue was dried under reduced pressure at 40°C to obtain compound (8) as a pale brown powder.
Actual yield: 4.90 g (percent yield: 65%)
¹H-NMR (DMSO-d₆) δ/ppm: 3.09 (t, 2H), 3.44 (t, 2H), 3.52 (t, 2H), 4.65-4.85 (m, 12H), 6.72-7.49 (m, 14H)

### Synthesis of Compound (7)

Compound (7) was obtained as a pale yellow powder in the same manner as for the synthesis of compound (8), except that compound (P-4) was used instead of compound (P-2).
Actual yield: 4.4 g (percent yield: 58%)
¹H-NMR (DMSO-d₆) δ/ppm: 3.09 (t, 2H), 3.48 (t, 2H), 3.71 (t, 2H), 4.60-4.75 (m, 4H), 4.86 (d, 4H), 5.04 (d, 4H), 6.66-7.53 (m, 14H)

### Synthesis of Compound (96)

6.64 g (15 mmol) of compound (P-7) and 60 g of dimethyl sulfoxide (DMSO) were placed in a reaction flask equipped with a reflux tube, and 2.75 g (33 mmol) of a 48% aqueous sodium hydroxide solution was added dropwise while stirring at room temperature under a nitrogen stream. Subsequently, 3.93 g (33 mmol) of 3-bromo-1-propyne was added dropwise, and the mixture was then stirred at room temperature for 2 hours. The reaction solution was poured into 350 g of water, and after stirring for 30 minutes, the precipitate was collected by filtering. 50 g of isopropanol was added to the collected filter cake, followed by stirring for 30 minutes. Then, the mixture was filtered, and the residue was dried under reduced pressure at 40°C to obtain compound (96) as a pale brown powder.
Actual yield: 4.3 g (percent yield: 55%)
¹H-NMR (DMSO-d₆) δ/ppm: 3.53 (t, 2H), 4.79 (d, 4H), 5.67 (s, 2H), 6.80-7.27 (m, 16H), 10.73 (s, 2H)

### Synthesis of Compound (2)

6.61 g (15 mmol) of compound (P-8) and 60 g of dimethyl sulfoxide (DMSO) were placed in a reaction flask equipped with a reflux tube, and 5.29 g (66 mmol) of a 48% aqueous sodium hydroxide solution was added dropwise while stirring at room temperature under a nitrogen stream. Subsequently, 7.55 g (63 mmol) of 3-bromo-1-propyne was added dropwise, and the mixture was then stirred at room temperature for 2 hours. The reaction solution was poured into 350 g of water, and after stirring for 30 minutes, the precipitate was collected by filtering. The collected filter cake was dissolved in 50 g of ethyl acetate, and then, 50 g of water was added. The mixture was stirred for 30 minutes, followed by oil/water separation. The solvent was removed from the organic layer, and the residue was dried under reduced pressure at 40°C to obtain compound (2) as a pale brown powder.
Actual yield: 3.8 g (percent yield: 42%)
¹H-NMR (DMSO-d₆) δ/ppm: 3.10 (t, 2H), 3.56 (t, 2H), 4.65 (d, 4H), 4.92 (d, 4H), 6.61-7.69 (m, 16H)

### Synthesis of Compound (3)

Compound (3) was obtained as a pale brown powder in the same manner as for the synthesis of compound (2), except that compound (P-6) was used instead of compound (P-8).
Actual yield: 4.1 g (percent yield: 45%)
¹H-NMR (DMSO-d₆) δ/ppm: 3.03 (t, 2H), 3.69 (t, 2H), 4.64 (d, 4H), 5.01 (d, 4H), 6.54 (d, 2H), 6.89 (t, 2H), 7.31-7.50 (m, 12H)

### Synthesis of Compound (36)

4.09 g (10 mmol) of 6,12-diphenyl -5,11-dihydroindolo[3,2-b]carbazole and 40 g of dimethyl sulfoxide (DMSO) were placed in a reaction flask equipped with a reflux tube, and 1.83 g (22 mmol) of a 48% aqueous sodium hydroxide solution was added dropwise while stirring at room temperature under a nitrogen stream. Subsequently, 2.50 g (21 mmol) of 3-bromo-1-propyne was added dropwise, and the mixture was then stirred at room temperature for 2 hours. The reaction solution was poured into 200 g of water, and after stirring for 30 minutes, the precipitate was collected by filtering. The collected filter cake was dissolved in 50 g of ethyl acetate, and then, 50 g of water was added. The mixture was stirred for 30 minutes, followed by oil/water separation. The solvent was removed from the organic layer, and the residue was dried under reduced pressure at 40°C to obtain compound (36) as a pale brown powder.
Actual yield: 2.3 g (percent yield: 47%)
¹H-NMR (DMSO-d₆) δ/ppm: 3.06 (t, 2H), 4.60 (d, 4H), 6.45-7.78 (m, 18H)

### Synthesis of Compound (76)

5.20 g (11 mmol) of compound (P-2) and 50 g of tetrahydrofuran (THF) were placed in a reaction flask equipped with a reflux tube, and 3.67 g (44 mmol) of a 48% aqueous sodium hydroxide solution was added dropwise while stirring at room temperature under a nitrogen stream. Subsequently, 5.00 g (42 mmol) of 3-bromo-1-propyne was added dropwise, and the mixture was then stirred at room temperature for 1 hour. The reaction solution was poured into 300 g of water, and after stirring for 30 minutes, the precipitate was collected by filtering. 50 g of isopropanol was added to the collected filter cake. Then, the mixture was stirred for 30 minutes and filtered, and the residue was dried under reduced pressure at 40°C to obtain compound (76) as a pale brown powder.
Actual yield: 1.3 g (percent yield: 20%)
¹H-NMR (DMSO-d₆) δ/ppm: 3.39 (t, 2H), 3.53 (t, 2H), 4.61 (d, 4H), 6.82 (t, 2H), 7.09-7.43 (m, 12H), 10.41 (s, 2H)

### Synthesis of Compound (42)

5.69 g (10 mmol) of compound (P-10) and 30 g of dimethyl sulfoxide (DMSO) were placed in a reaction flask equipped with a reflux tube, and 2.50 g (30 mmol) of a 48% aqueous sodium hydroxide solution was added dropwise while stirring at room temperature under a nitrogen stream. Subsequently, 3.54 g (30 mmol) of 3-bromo-1-propyne was added dropwise, and the mixture was then stirred at room temperature for 2 hours. The reaction solution was poured into 200 g of water, and after stirring for 30 minutes, the precipitate was collected by filtering. The collected filter cake was dissolved in 30 g of ethyl acetate, and then, 30 g of water was added. The mixture was stirred for 30 minutes, followed by oil/water separation. The solvent was removed from the organic layer, and the residue was dried under reduced pressure at 40°C to obtain compound (42) as a brown powder.
Actual yield: 4.0 g (percent yield: 63%)
¹H-NMR (DMSO-d₆) δ/ppm: 3.24 (t, 2H), 3.83 (s, 6H), 4.78 (d, 4H), 6.75-8.49 (m, 20H)

### Synthesis of Compound (81)

11.7 g (100 mmol) of indole, 11.2 g (100 mmol) of 3-thiophenecarboxyaldehyde, and 52 g of acetonitrile were placed in a reaction flask equipped with a reflux tube and stirred until completely dissolved. After water-cooling, 1.7 g (10 mmol) of 48% hydrobromic acid was slowly added dropwise. The reaction solution was returned to room temperature, and then stirred for 2 hours. The precipitate was collected by filtering and washed with 20 g of acetonitrile, and the filter cake was dried under reduced pressure at 40°C to obtain compound (P-11) as a pale yellow powder.
Actual yield: 13.1 g (percent yield: 62.1%)
¹H-NMR (DMSO-d₆) δ/ppm: 5.81 (s, 2H), 6.68-7.62 (s, 14H), 10.75 (s, 2H)

Then, 2.11 g (5.0 mmol) of compound (P-11) and 20 g of dimethyl sulfoxide (DMSO) were placed in a reaction flask equipped with a reflux tube, and 0.88 g (10.5 mmol) of a 48% aqueous sodium hydroxide solution was added dropwise while stirring at room temperature under a nitrogen stream. Subsequently, 1.24 g (10.5 mmol) of 3-bromo-1-propyne was added dropwise, and the mixture was then stirred at room temperature for 3 hours. The reaction solution was poured into 50 g of water, and after stirring for 30 minutes, the precipitate was collected by filtering. 20 g of ethyl acetate was added to the collected filter cake, followed by stirring for 30 minutes. Then, the mixture was filtered, and the filter cake was dried under reduced pressure at 40°C to obtain compound (81) as a pale yellow powder.
Actual yield: 1.8 g (percent yield: 72.3%)
¹H-NMR (DMSO-d₆) δ/ppm: 3.11 (t, 2H), 4.68 (d, 2H), 5.08 (d, 2H), 6.11 (s, 2H), 6.60-7.81 (m, 14H)

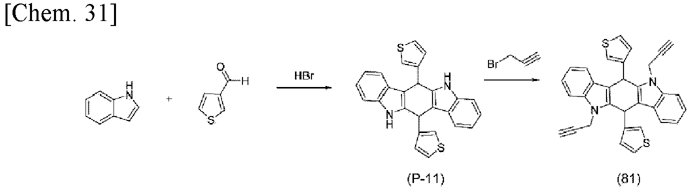

### Synthesis of Compound (46)

2.2 g (19 mmol) of indole, 2.4 g (19 mmol) of 4-ethynylbenzaldehyde, and 13 g of acetonitrile were placed in a reaction flask equipped with a reflux tube and stirred until completely dissolved. After water-cooling, 0.6 g (3.8 mmol) of 48% hydrobromic acid was slowly added dropwise. The reaction solution was returned to room temperature, and then stirred for 2 hours. The reaction solution was concentrated, and 9 g of toluene was added. The mixture was then cooled to 10°C and added dropwise to 36 g of hexane. The precipitate was collected by filtering, and the filter cake was dried under reduced pressure at 40°C to obtain compound (46) as a light yellow powder.
Actual yield: 3.6 g (percent yield: 83.6%)
¹H-NMR (DMSO-d₆) δ/ppm: 2.30 (s, 2H), 4.11 (s, 2H), 6.80-7.84 (m, 16H), 10.78 (s, 2H)

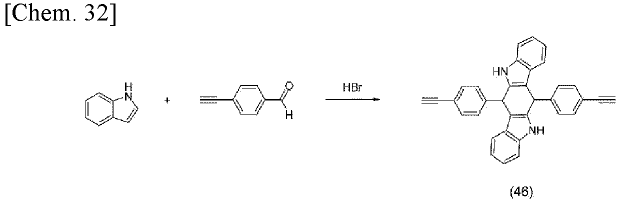

### Synthesis of Compound (53)

11.7 g (100 mmol) of indole, 19.4 g (100 mmol) of 2-fluorenecarboxyaldehyde, and 90 g of acetonitrile were placed in a reaction flask equipped with a reflux tube and stirred until completely dissolved. After water-cooling, 1.7 g (10 mmol) of 48% hydrobromic acid was slowly added dropwise. The reaction solution was returned to room temperature, and then stirred for 2 hours. The reaction solution was cooled to 10°C, and the precipitate was collected by filtering and washed with 20 g of acetonitrile. The filter cake was dried under reduced pressure at 40°C to obtain compound (P-12) as a brown powder.
Actual yield: 27.0 g (percent yield: 92.1%)
¹H-NMR (DMSO-d₆) δ/ppm: 3.84 (s, 4H), 5.85 (s, 2H), 6.77-7.87 (m, 22H), 10.76 (s, 2H)

Then, 5.87 g (10.0 mmol) of compound (P-12) and 30 g of dimethyl sulfoxide (DMSO) were placed in a reaction flask equipped with a reflux tube, and 1.83 g (22.0 mmol) of a 48% aqueous sodium hydroxide solution was added dropwise while stirring at room temperature under a nitrogen stream. Subsequently, 2.59 g (22.0 mmol) of 3-bromo-1-propyne was added dropwise, and the mixture was then stirred at room temperature for 3 hours. The reaction solution was poured into 100 g of water, and after stirring for 30 minutes, the precipitate was collected by filtering. 30 g of ethyl acetate was added to the collected filter cake, followed by stirring for 30 minutes. Then, the mixture was filtered, and the filter cake was dried under reduced pressure at 40°C to obtain compound (53) as a pale orange powder.
Actual yield: 3.2 g (percent yield: 48%)
¹H-NMR (DMSO-d₆) δ/ppm: 2.94 (t, 2H), 3.86 (s, 4H), 4.72 (d, 2H), 4.92 (d, 2H), 6.10 (s, 2H), 6.91-8.30 (m, 22H)

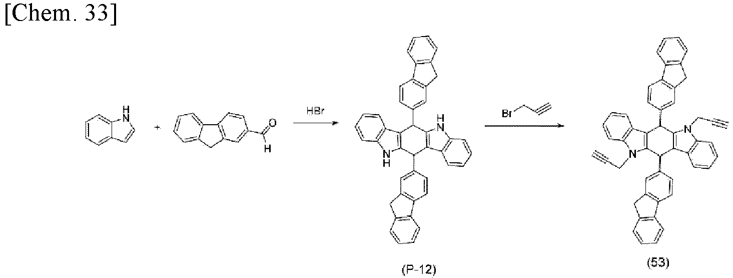

### Synthesis of Compound (51)

2.35 g (4.0 mmol) of compound (P-12) and 30 g of dimethyl sulfoxide (DMSO) were placed in a reaction flask equipped with a reflux tube, and 3.0 g (36.0 mmol) of a 48% aqueous sodium hydroxide solution was added dropwise while stirring at room temperature under a nitrogen stream. Subsequently, 4.25 g (36.0 mmol) of 3-bromo-1-propyne was added dropwise, and the mixture was then stirred at room temperature for 3 hours. The reaction solution was poured into 120 g of water, and after stirring for 30 minutes, the precipitate was collected by filtering. 20 g of methanol was added to the collected filter cake, followed by stirring for 30 minutes. Then, the mixture was filtered, and the filter cake was dried under reduced pressure at 40°C to obtain compound (51) as a pale orange powder.
Actual yield: 1.6 g (percent yield: 49%)
¹H-NMR (DMSO-d₆) δ/ppm: 2.47 (d, 8H), 2.94 (t, 4H), 3.05 (t, 2H), 4.72 (d, 2H), 4.92 (d, 2H), 6.10 (s, 2H), 6.90-8.34 (m, 22H)

### Synthesis of Compound (87)

11.7 g (100 mmol) of indole, 12.4 g (100 mmol) of 4-fluorobenzaldehyde, and 45 g of acetonitrile were placed in a reaction flask equipped with a reflux tube and stirred until completely dissolved. After water-cooling, 1.7 g (10 mmol) of 48% hydrobromic acid was slowly added dropwise. The reaction solution was returned to room temperature, and then stirred for 2 hours. The reaction solution was cooled to 10°C, and the precipitate was collected by filtering and washed with 20 g of acetonitrile. The filter cake was dried under reduced pressure at 40°C to obtain compound (P-13) as a gray powder.
Actual yield: 10.4 g (percent yield: 46.6%)
¹H-NMR (DMSO-d₆) δ/ppm: 5.74 (s, 2H), 6.80-7.38 (m, 16H), 10.75 (s, 2H)

Then, 2.23 g (5.0 mmol) of compound (P-13) and 15 g of dimethyl sulfoxide (DMSO) were placed in a flask equipped with a reflux tube, and 1.08 g (13.0 mmol) of a 48% aqueous sodium hydroxide solution was added dropwise while stirring at room temperature under a nitrogen stream. Subsequently, 1.53 g (13.0 mmol) of 3-bromo-1-propyne was added dropwise, and the mixture was then stirred at room temperature for 2 hours. The reaction solution was poured into 100 g of water, and after stirring for 30 minutes, the precipitate was collected by filtering. 30 g of methanol was added to the collected filter cake, followed by stirring for 30 minutes. The resulting suspension was filtered, and the filter cake was dried under reduced pressure at 40°C to obtain compound (87) as a pale yellow powder.
Actual yield: 1.3 g (percent yield: 50%)
¹H-NMR (DMSO-d₆) δ/ppm: 3.03 (t, 2H), 4.67 (d, 2H), 5.02 (d, 2H), 6.05 (s, 2H), 6.97-7.45 (m, 16H)

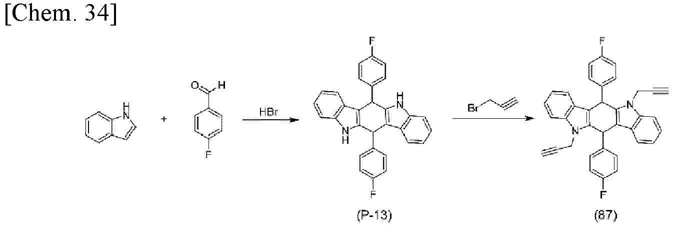

### Synthesis of Compound (94)

11.7 g (100 mmol) of indole, 13.1 g (100 mmol) of 3-formylbenzonitrile, and 50 g of acetonitrile were placed in a reaction flask equipped with a reflux tube and stirred until completely dissolved. After water-cooling, 1.7 g (10 mmol) of 48% hydrobromic acid was slowly added dropwise. The reaction solution was returned to room temperature, and then stirred for 2 hours. The reaction solution was cooled to 10°C, and the precipitate was collected by filtering and washed with 20 g of acetonitrile. The filter cake was dried under reduced pressure at 40°C to obtain compound (P-14) as a gray powder.
Actual yield: 3.8 g (percent yield: 16.5%)
¹H-NMR (DMSO-d₆) δ/ppm: 5.89 (s, 2H), 6.83-8.08 (m, 16H), 10.91 (s, 2H)

Then, 2.12 g (4.6 mmol) of compound (P-14) and 20 g of dimethyl sulfoxide (DMSO) were placed in a flask equipped with a reflux tube, and 1.00 g (12.0 mmol) of a 48% aqueous sodium hydroxide solution was added dropwise while stirring at room temperature under a nitrogen stream. Subsequently, 1.41 g (12.0 mmol) of 3-bromo-1-propyne was added dropwise, and the mixture was then stirred at room temperature for 2 hours. The reaction solution was poured into 120 g of water, and after stirring for 30 minutes, the precipitate was collected by filtering. 40 g of methanol was added to the collected filter cake, followed by stirring for 30 minutes. The resulting suspension was filtered, and the filter cake was dried under reduced pressure at 40°C to obtain compound (94) a pale orange powder.
Actual yield: 1.5 g (percent yield: 61%)
¹H-NMR (DMSO-d₆) δ/ppm: 2.89 (t, 2H), 4.81 (d, 2H), 5.03 (d, 2H), 6.19 (s, 2H), 7.00-7.94 (m, 16H)

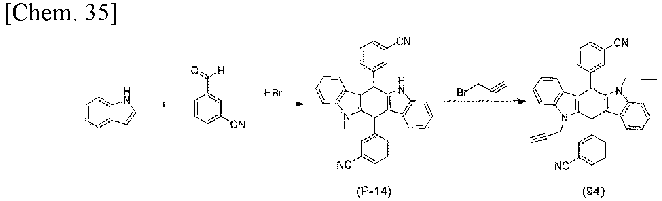

### Synthesis of Compound (45)

30.0 g (256 mmol) of indole, 52.0 g (256 mmol) of 4-(trimethylsilyl)ethynylbenzaldehyde, and 230 g of acetonitrile were placed in a reaction flask equipped with a reflux tube, and 9.0 g (52.0 mmol) of 48% hydrobromic acid was slowly added dropwise while stirring under water-cooling. The reaction solution was returned to room temperature, and then stirred for 2 hours. The reaction solution was cooled to 10°C, and the precipitate was collected by filtering and washed with 100 g of acetonitrile. The filter cake was dried under reduced pressure at 40°C to obtain compound (P-15) as a pale yellow powder.
Actual yield: 25.0 g (percent yield: 33%)

Then, 22.0 g (36 mmol) of compound (P-15), 10.0 g (40 mmol) of chloranil, and 880 g of o-xylene were placed in a reaction flask equipped with a reflux tube, the temperature was raised, and a reaction was carried out at 100°C for 30 hours. The reaction solution was cooled to room temperature, and the solvent was removed. Then, 200 g of methanol was added to the residue, and the mixture was stirred at room temperature for 30 minutes. The precipitate was collected by filtering and washed twice with 200 g of methanol, and the filter cake was dried under reduced pressure at 40°C to obtain compound (P-16) as a pale yellow powder.
Actual yield: 10.9 g (percent yield: 50%)

Then, 9.0 g (15 mmol) of compound (P-16), 80 g of tetrahydrofuran, and 80 g of methanol were placed in a reaction flask equipped with a reflux tube and stirred at room temperature for 30 minutes. Then, 2.3 g (16 mmol) of potassium carbonate was added, and a reaction was carried out at room temperature for 3 hours. 20 g of ion-exchanged water was added to the reaction solution, and the precipitate was collected by filtering. The filter cake was washed three times with 20 g of methanol and then dried under reduced pressure at 40°C to obtain compound (45) as a pale yellow powder.
Actual yield: 6.1 g (percent yield: 88%)
¹H-NMR (DMSO-d₆) δ/ppm: 4.37 (s, 2H), 6.88-7.83 (m, 16H), 10.64 (s, 2H)

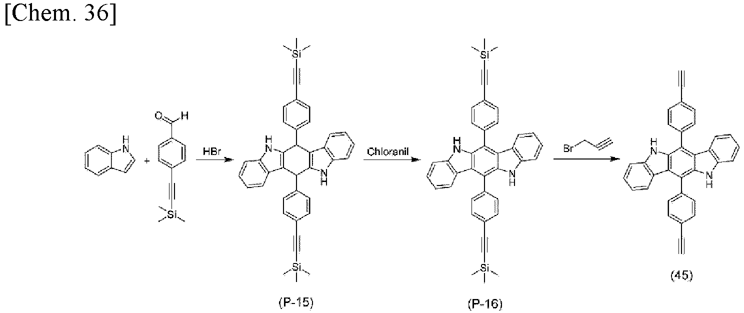

### Synthesis of Compound (88)

2.23 g (5.0 mmol) of compound (P-13) and 15 g of dimethyl sulfoxide (DMSO) were placed in a flask equipped with a reflux tube, and 1.08 g (13.0 mmol) of a 48% aqueous sodium hydroxide solution was added dropwise under stirring at room temperature under a nitrogen stream. Subsequently, 1.57 g (13.0 mmol) of 3-bromo-1-propene was added dropwise, and the mixture was then stirred at room temperature for 1 hour. The reaction solution was poured into 100 g of water, and after stirring for 30 minutes, the precipitate was collected by filtering. 30 g of methanol was added to the collected filter cake, followed by stirring for 30 minutes. The resulting suspension was filtered, and the filter cake was dried under reduced pressure at 40°C to obtain compound (88) as a pale yellow powder.
Actual yield: 1.9 g (percent yield: 72%)
¹H-NMR (DMSO-d₆) δ/ppm: 4.45 (m, 2H), 4.70-4.92 (m, 6H), 5.26 (m, 2H), 5.97 (s, 2H), 6.90-7.43 (m, 16H)

### Synthesis of Compound (95)

3.10 g (7.0 mmol) of compound (P-7) and 20 g of dimethyl sulfoxide (DMSO) were placed in a flask equipped with a reflux tube, and 2.92 g (35.0 mmol) of a 48% aqueous sodium hydroxide solution was added dropwise while stirring at room temperature under a nitrogen stream. Subsequently, 4.23 g (35.0 mmol) of 3-bromo-1-propene was added dropwise, and the mixture was then stirred at room temperature for 3 hours. The reaction solution was poured into 100 g of water, and after stirring for 30 minutes, the precipitate was collected by filtering. 30 g of methanol was added to the collected filter cake, followed by stirring for 30 minutes. The resulting suspension was filtered, and the filter cake was dried under reduced pressure at 40°C to obtain compound (95) as a pale yellow powder.
Actual yield: 3.0 g (percent yield: 71%)
¹H-NMR (DMSO-d₆) δ/ppm: 4.52 (m, 4H), 4.89 (m, 4H), 5.17-5.34 (m, 10H), 5.89 (s, 2H), 5.96 (m, 2H), 6.72-7.46 (m, 16H)

### Synthesis of Compound (97)

13.1 g (100 mmol) of 1-methylindole, 12.2 g (100 mmol) of 3-hydroxybenzaldehyde, and 60 g of acetonitrile were placed in a reaction flask equipped with a reflux tube and stirred until completely dissolved. After water-cooling, 1.7 g (10 mmol) of 48% hydrobromic acid was slowly added dropwise. After it was confirmed that heat generation had subsided, the mixture was returned to room temperature and then stirred for 1 hour. The precipitate in the reaction solution was collected by filtering and washed twice with 20 g of acetonitrile, and the filter cake was dried under reduced pressure at 40°C to obtain compound (P-17) as a pinkish gray powder.
Actual yield: 8.1 g (percent yield: 35%)

Then, 4.70 g (10 mmol) of compound (P-17), 20 g of tetrahydrofuran (THF), and 2.42 g (24 mmol) of triethylamine were placed in a reaction flask equipped with a reflux tube. Then, 2.18 g (24 mmol) of acrylic acid chloride was added dropwise while stirring at room temperature under a nitrogen stream, and the mixture was then stirred for 30 minutes. The temperature was raised to 60°C, and the reaction was continued at that temperature for 3 hours, followed by cooling to room temperature. 20 g of THF was added, and the resulting mixture was stirred for 10 minutes. Then, the precipitate was filtered off, and the solvent was removed from the filtrate. Next, 20 g of 1% hydrochloric acid was added to the residue, and the mixture was stirred for 15 minutes and then filtered. The filter cake was washed twice with 20 g of ion-exchanged water and then washed with 20 g of methanol, and the resulting filter cake was dried under reduced pressure at 40°C to obtain compound (97) as a pale orange powder.
Actual yield: 1.5 g (percent yield: 52%)
¹H-NMR (DMSO-d₆) δ/ppm: 3.37 (s, 6H), 5.92 (s, 2H), 6.11-6.53 (m, 6H), 6.93-7.69 (m, 16H)

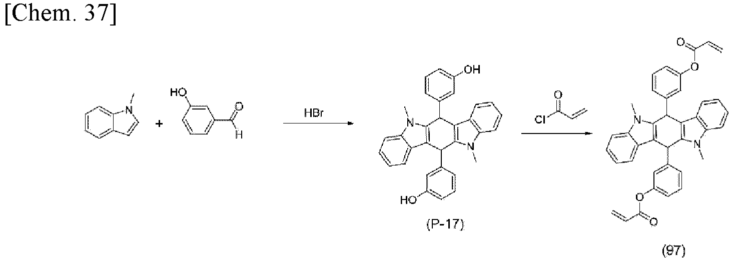

### Synthesis of Compound (44)

2.0 g (4.0 mmol) of compound (45) and 100 g of dimethyl sulfoxide (DMSO) were placed in a flask equipped with a reflux tube, and 2.1 g (18 mmol) of 3-bromo-1-propene was added dropwise while stirring at room temperature under a nitrogen stream. Subsequently, 1.1 g (13 mmol) of a 48% aqueous sodium hydroxide solution was added, and the mixture was then stirred at room temperature for 1 hour. Then, 200 g of water was added dropwise to the reaction solution, and after stirring for 30 minutes, the precipitate was collected by filtering. 50g of methanol and 100 g of water were added to the collected filter cake, and the mixture was stirred for 30 minutes. The resulting suspension was filtered, and the filter cake was dried under reduced pressure at 40°C to obtain compound (44) as a pale yellow powder.
Actual yield: 2.1 g (percent yield: 91%)
¹H-NMR (DMSO-d₆) δ/ppm: 3.08 (t, 2H), 4.43 (s, 2H), 4.60 (d, 4H), 6.91-7.84 (m, 16H)

### Synthesis of Compound (50)

15.0 g (26 mmol) of compound (P-12) and 450 g of o-xylene were placed in a flask equipped with a reflux tube, 7.8 g (31 mmol) of iodine was added under stirring, and the reaction was carried out at 140°C for 5 hours.

The reaction solution was cooled to room temperature, 120 g of a 10% aqueous sodium thiosulfate solution was added dropwise, and the mixture was stirred for 30 minutes. The precipitate was collected by filtering. Then, the filter cake was washed with 50 g of o-xylene and further washed twice with 100 g of a mixed solvent of water/methanol = 1/2 (weight ratio). The resulting filter cake was dried under reduced pressure at 40°C to obtain compound (P-18) as a gray powder.
Actual yield: 13.3 g (percent yield: 89%)
¹H-NMR (THF-ds) δ/ppm: 4.12 (s, 4H), 6.77-8.19 (m, 22H), 9.72 (s, 2H)

Then, 5.0 g (9.0 mmol) of compound (P-18) and 250 g of dimethyl sulfoxide (DMSO) were placed in a reaction flask equipped with a reflux tube, and 9.0 g (77 mmol) of a 48% aqueous potassium hydroxide solution was added dropwise while stirring at room temperature under a nitrogen stream. Subsequently, 9.2 g (77 mmol) of 3-bromo-1-propyne was added dropwise, and the mixture was then stirred at room temperature for 1 hour. The reaction solution was poured into 250 g of water, and the mixture was stirred for 30 minutes and filtered. 250 g of water was added to the filtrate, the mixture was stirred for 30 minutes and then filtered. The filter cake was washed twice with 100 g of a mixed solvent of water/methanol = 1/2 (weight ratio). The filter cake was dried under reduced pressure at 40°C to obtain compound (50) as a yellow powder.
Actual yield: 2.2 g (percent yield: 44%)
¹H-NMR(THF-d₈) δ/ppm: 2.51 (d, 8H), 2.63 (t, 4H), 3.05 (t, 2H), 4.67 (d, 4H), 6.76-8.19 (m, 22H)

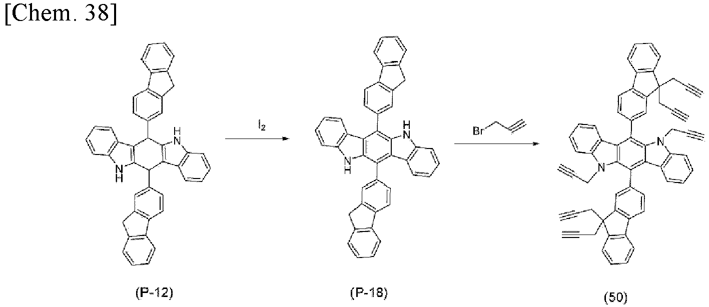

### Synthesis of Compound (82)

9.4 g (80 mmol) of indole, 13.0 g (80 mmol) of benzo[b]thiophene-3-carboxyaldehyde, and 65 g of acetonitrile were placed in a reaction flask equipped with a reflux tube and stirred until completely dissolved. After water-cooling, 1.4 g (8 mmol) of 48% hydrobromic acid was slowly added dropwise. After it was confirmed that heat generation had subsided, the mixture was returned to room temperature and then stirred for 2 hours. The precipitate in the reaction solution was collected by filtering and washed with 50 g of acetonitrile, and the filter cake was dried under reduced pressure at 40°C to obtain compound (P-19) as a brown powder.
Actual yield: 12.2 g (percent yield: 58%)
¹H-NMR (DMSO-d₆) δ/ppm: 6.36 (s, 2H), 6.75-7.31 (m, 14H), 7.91-8.24 (m, 4H), 10.84 (s, 2H)

Then, 10.5 g (20 mmol) of compound (P-19) and 120 g of dimethyl sulfoxide (DMSO) were placed in a reaction flask equipped with a reflux tube, and 4.3 g (52 mmol) of a 48% aqueous potassium hydroxide solution was added dropwise while stirring at room temperature under a nitrogen stream. Subsequently, 6.2 g (52 mmol) of 3-bromo-1-propyne was added dropwise, and the mixture was then stirred at room temperature for 2 hours. The reaction solution was poured into 300 g of water, and the mixture was stirred for 30 minutes and filtered. 300 g of methanol was added to the filter cake, and the mixture was then stirred for 30 minutes and filtered. The filter cake was dried under reduced pressure at 40°C to obtain compound (82) as a pale yellow powder.
Actual yield: 8.5 g (percent yield: 71%)
¹H-NMR (DMSO-d₆) δ/ppm: 2.97 (t, 2H), 4.77 (d, 2H), 5.14 (d, 2H), 6.64 (s, 2H), 6.88-7.92 (m, 18H)

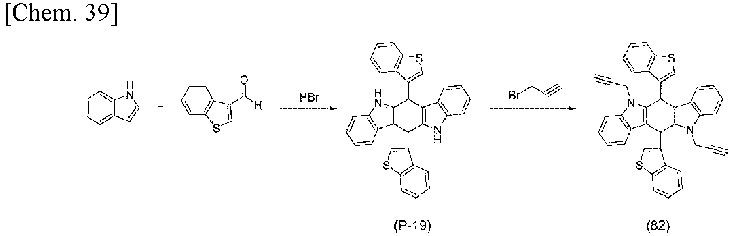

### Synthesis of Compound (101)

4.0 g (30 mmol) of 5-hydroxyindole, 5.8 g (30 mmol) of 2-fluorenecarboxyaldehyde, and 30 g of acetonitrile were placed in a flask equipped with a reflux tube. 0.51 g (3 mmol) of 48% hydrobromic acid was added dropwise at room temperature under stirring, and the mixture was stirred for 2 hours. 10 g of water was added to the reaction solution, and the mixture was stirred at room temperature for 30 minutes and then filtered. The collected filter cake was washed three times with 10 g of methanol and then dried under reduced pressure at 40°C to obtain compound (P-20) as a greenish gray powder.
Actual yield: 5.2 g (percent yield: 56%)
¹H-NMR (DMSO-d₆) δ/ppm: 4.12 (s, 4H), 5.66 (s, 2H), 6.13-8.29 (m, 20H), 10.09 (s, 2H), 10.33 (s, 2H)

Then, 4.5 g (7 mmol) of compound (P-20) and 90 g of acetonitrile were placed in a flask equipped with a reflux tube, and 2.2 g (9 mmol) of chloranil was added under stirring at room temperature. The mixture was stirred at room temperature for 30 minutes, the temperature was then raised to 70°C, and the mixture was heated and stirred at that temperature for 7 hours. The reaction solution was cooled to room temperature and then filtered. The collected filter cake was washed six times with 30 g of o-xylene and then dried under reduced pressure at 40°C to obtain compound (P-21) as a gray powder.
Actual yield: 4.5 g (percent yield: 100%)
¹H-NMR (DMSO-d₆) δ/ppm: 4.12 (s, 4H), 6.43-8.24 (m, 20H), 10.09 (s, 2H), 10.33 (s, 2H)

Then, 4.0 g (6 mmol) of compound (P-21) and 60 g of dimethyl sulfoxide (DMSO) were placed in a reaction flask equipped with a reflux tube, and 4.3 g (52 mmol) of a 48% aqueous potassium hydroxide solution was added while stirring at room temperature under a nitrogen stream. Subsequently, 6.2 g (52 mmol) of 3-bromo-1-propene was added, and the mixture was then stirred at room temperature for 1 hour. Then, 80 g of water was added dropwise to the reaction solution, and after stirring for 30 minutes, the precipitate was collected by filtering. The collected filter cake was washed with 10 g of a mixed solvent of methanol/water = 1/1 and then dried under reduced pressure at 40°C to obtain compound (101) as a reddish brown powder.
Actual yield: 3.7 g (percent yield: 61%)
¹H-NMR (DMSO-d₆) δ/ppm: 2.55 (d, 8H), 2.73 (t, 4H), 3.05 (t, 2H), 3.38 (t, 2H), 4.65 (d, 4H), 4.74 (d, 4H), 6.74-8.26 (m, 20H)

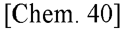

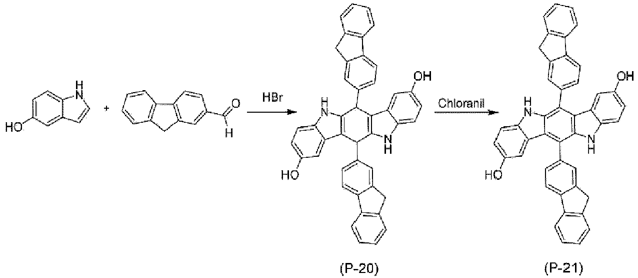

### Synthesis of Compound (105)

23.4 g (200 mmol) of indole, 30.0 g (200 mmol) of piperonal, and 150 g of acetonitrile were placed in a reaction flask equipped with a reflux tube and stirred until completely dissolved. After water-cooling, 3.4 g (20 mmol) of 48% hydrobromic acid was slowly added dropwise. After it was confirmed that heat generation had subsided, the mixture was returned to room temperature and then stirred for 3 hours. The precipitate in the reaction solution was collected by filtering and washed with 100 g of acetonitrile, and the filter cake was dried under reduced pressure at 40°C to obtain compound (P-22) as a pale pink powder.
Actual yield: 37.0 g (percent yield: 74%)
¹H-NMR (DMSO-d₆) δ/ppm: 5.63 (s, 2H), 5.93 (s, 4H), 6.72-7.27 (m, 14H), 10.68 (s, 2H)

Then, 25.0 g (50 mmol) of compound (P-22), 13.5 g (55 mmol) of chloranil, and 375 g of o-xylene were placed in a reaction flask equipped with a reflux tube, the temperature was raised, and a reaction was carried out at 130°C for 9 hours. The reaction solution was cooled to room temperature, and the precipitate was collected by filtering. 300 g of methanol was added to the filter cake, and the mixture was stirred at room temperature for 30 minutes and filtered. The filter cake was dried under reduced pressure at 40°C to obtain compound (P-23) as a brown powder.
Actual yield: 21.1 g (percent yield: 84%)
¹H-NMR (DMSO-d₆) δ/ppm: 6.21 (s, 4H), 6.87-7.46 (m, 14H), 10.54 (s, 2H)

Then, 15.0 g (30 mmol) of compound (P-22) and 150 g of dimethyl sulfoxide (DMSO) were placed in a reaction flask equipped with a reflux tube, and 6.5 g (78 mmol) of a 48% aqueous potassium hydroxide solution was added dropwise while stirring at room temperature under a nitrogen stream. Subsequently, 9.3 g (78 mmol) of 3-bromo-1-propyne was added dropwise, and the mixture was then stirred at room temperature for 2 hours. The reaction solution was poured into 300 g of water, and the mixture was stirred for 30 minutes and filtered. 250 g of water was added to the filter cake, and the mixture was stirred for 30 minutes and then filtered. Furthermore, the filter cake was washed twice with 100 g of a mixed solvent of water/2-propanol = 1/1 (weight ratio). The resulting filter cake was dried under reduced pressure at 40°C to obtain compound (105) as a pale brown powder.
Actual yield: 15.8 g (percent yield: 93%)
¹H-NMR (DMSO-d₆) δ/ppm: 3.08 (t, 2H), 4.71 (d, 4H), 6.26 (s, 4H), 6.72-7.54 (m, 14H)

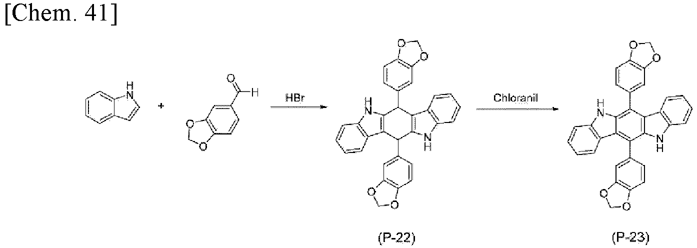

### Synthesis of Compound (115)

9.3 g (80 mmol) of indole, 22.7 g (80 mmol) of 9-benzyl-9H-carbazole-3-carboxyaldehyde, and 150 g of acetonitrile were placed in a reaction flask equipped with a reflux tube, and the temperature of the mixture was raised to 45°C under stirring. After it was confirmed that the components had dissolved and formed a uniform solution, 1.3 g (8 mmol) of 48% hydrobromic acid was slowly added dropwise. After it was confirmed that heat generation had subsided, the mixture was returned to room temperature and then stirred for 3 hours. The precipitate in the reaction solution was collected by filtering and washed with 100 g of acetonitrile, and the filter cake was dried under reduced pressure at 40°C to obtain compound (P-24) as a reddish brown powder.
Actual yield: 24.1 g (percent yield: 79%)
¹H-NMR (DMSO-d₆) δ/ppm: 5.61 (s, 4H), 5.94 (s, 2H), 6.71-8.30 (m, 32H), 10.70 (s, 2H)

Then, 20.0 g (26 mmol) of compound (P-24) and 200 g of dimethyl sulfoxide (DMSO) were placed in a flask equipped with a reflux tube, and 4.8 g (57 mmol) of a 48% aqueous sodium hydroxide solution was added dropwise while stirring at room temperature under a nitrogen stream. Subsequently, 14.2 g (57 mmol) of 3-bromo-1-propene was added dropwise, and the mixture was then stirred at room temperature for 1 hour. Then, 1500 g of water was added dropwise to the reaction solution, and after stirring for 30 minutes, the precipitate was collected by filtering. 100g of methanol and 200 g of water were added to the collected filter cake, and the mixture was stirred for 30 minutes. The resulting suspension was filtered, and the filter cake was dried under reduced pressure at 40°C to obtain compound (115) as a pale yellow powder.
Actual yield: 20.0 g (percent yield: 91%)
¹H-NMR (DMSO-d₆) δ/ppm: 3.02 (t, 2H), 4.63 (d, 4H), 5.62 (s, 4H), 5.95 (s, 2H), 6.73-8.35 (m, 32H)

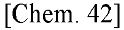

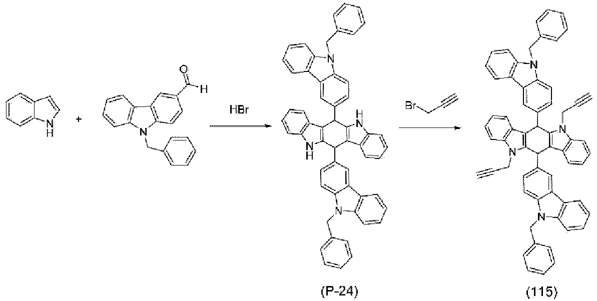

### Synthesis of Compound (119)

9.1 g (62 mmol) of 5-methoxyindole, 12.0 g (62 mmol) of fluorene-2-carboxyaldehyde, and 40 g of acetonitrile were placed in a reaction flask equipped with a reflux tube and stirred until completely dissolved. After water-cooling, 0.5 g (3 mmol) of 48% hydrobromic acid was slowly added dropwise. After it was confirmed that heat generation had subsided, the mixture was returned to room temperature and then stirred for 3 hours. The precipitate in the reaction solution was collected by filtering and washed with 100 g of acetonitrile, and the filter cake was dried under reduced pressure at 40°C to obtain compound (P-25) as a grayish green powder.
Actual yield: 14.5 g (percent yield: 73%)
¹H-NMR(THF-d₈) δ/ppm: 3.47 (s, 6H), 3.83 (s, 4H), 5.79 (s, 2H), 6.57-8.12 (m, 20H), 9.70 (s, 2H)

Then, 12.5 g (19 mmol) of compound (P-25), 5.0 g (mmol) of chloranil, and 50 g of dimethylformamide were placed in a reaction flask equipped with a reflux tube, the temperature was raised, and a reaction was carried out at 100°C for 2 hours. The reaction solution was cooled to room temperature, and the precipitate was collected by filtering. 300 g of methanol was added to the filter cake, and the mixture was stirred at room temperature for 30 minutes and filtered. The filter cake was dried under reduced pressure at 40°C to obtain compound (P-26) as a grayish green powder.
Actual yield: 8.0 g (percent yield: 64%)
¹H-NMR(THF-d₈) δ/ppm: 3.47 (s, 6H), 4.14 (s, 4H), 6.89-8.18 (m, 20H), 9.49 (s, 2H)

Then, 6.5 g (10 mmol) of compound (P-26) and 130 g of dimethyl sulfoxide (DMSO) were placed in a reaction flask equipped with a reflux tube, and 7.6 g (91 mmol) of a 48% aqueous potassium hydroxide solution was added dropwise while stirring at room temperature under a nitrogen stream. Subsequently, 22.5 g (91 mmol) of 3-bromo-1-propyne was added dropwise, and the mixture was then stirred at room temperature for 3 hours. The reaction solution was poured into 1000 g of water, and the mixture was stirred for 30 minutes and filtered. 200 g of water was added to the filter cake, and the mixture was stirred for 30 minutes and then filtered. Furthermore, the filter cake was washed twice with 100 g of a mixed solvent of water/2-propanol = 1/1 (weight ratio). The resulting filter cake was dried under reduced pressure at 40°C to obtain compound (119) a pale brown powder.
Actual yield: 6.3 g (percent yield: 72%)
¹H-NMR (THF-ds) δ/ppm: 2.54 (d, 8H), 2.65 (t, 4H), 3.07 (t, 2H), 3.49 (s, 6H), 4.69 (d, 4H), 6.89-8.18 (m, 20H)

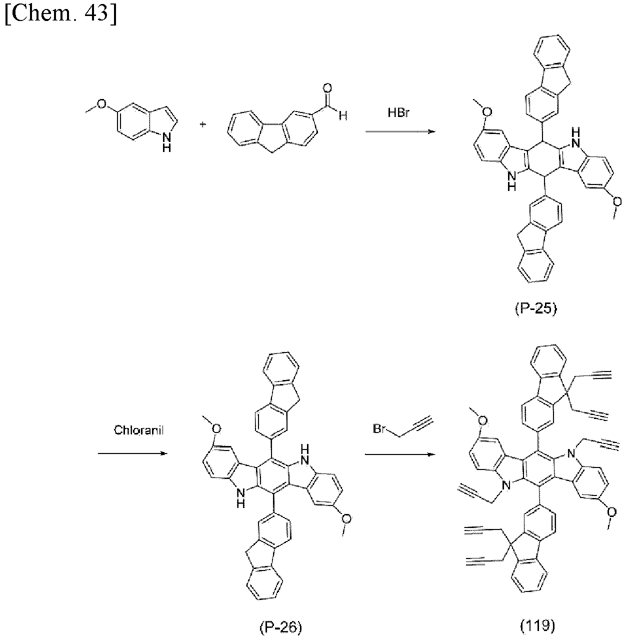

### Examples 1 to 23 and Comparative Examples 1 to 6

Components were weighed according to the formulation (parts by mass) shown in Table 1, and they were mixed with a solvent and then dissolved through stirring. After it was confirmed that all solids were completely dissolved, the solution was filtered through a PTFE filter (pore size: 0.45 µm) to obtain a composition for evaluation.

The components shown in the table were as follows.

| | | | |
|---|---|---|---|
| A1: | Compound (2) | | Compound (I) |
| A2: | Compound (94) | | Compound (I) |
| A3: | Compound (8) | | Compound (I) |
| A4: | Compound (13) | | Compound (I) |
| A5: | Compound (76) | | Compound (I) |

| | | | |
|---|---|---|---|
| A6: | Compound (36) | Compound (I) | |
| A7: | Compound (45) | Compound (I) | |
| A8: | Compound (88) | Compound (I) | |
| A9: | Compound (53) | Compound (I) | |
| A10: | Compound (A10) below | | Compound with a specific skeleton, but without reactive group |
| A11: | Compound (A11) below | | Fluorene compound having a reactive group |
| A12: | Compound (A12) below | | Fluorene compound having a reactive group |
| A13: | Compound (44) | Compound (I) | |
| A14: | Compound (50) | Compound (I) | |
| A15: | Compound (101) | Compound (I) | |
| B1: | Compound (B1) below | | Cross-linking agent (Glycoluril compound) |
| B2: | Compound (B2) below | | Cross-linking agent (Phenolic compound, but not the compound (I)) |
| B3: | Compound (B3) below | | Cross-linking agent (Phenolic compound, but not the compound (I)) |
| C1: | Bis(4-tert-butylphenyl)iodonium nonafluorobutanesulfonate (Polymerization initiator: Thermal acid generator) | | |
| D1: | Propylene glycol monomethyl ether acetate (PGMEA) solvent | | |

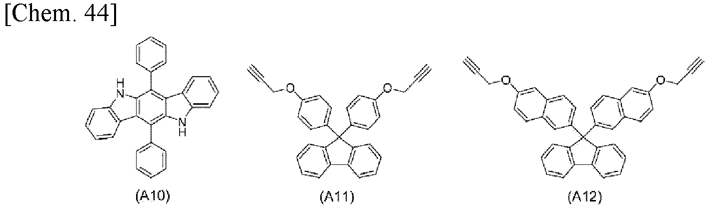

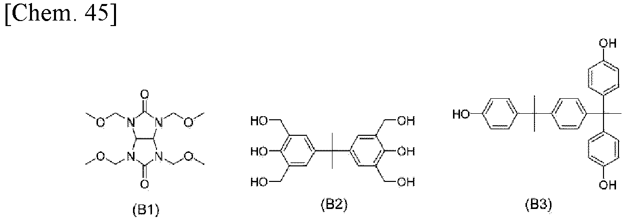

The prepared composition for evaluation was applied to a glass substrate (EAGLE XG manufactured by Corning Incorporated) using a spin coater so as to achieve a film thickness of 1.0 µm after heating. The coated substrate was heated on a hot plate at 170°C for 120 seconds and then further heated on a hot plate at 300°C for 120 seconds, to obtain a substrate for evaluation.

On the substrate for evaluation, the film condition, heat resistance, and solvent resistance were evaluated in the following manner. Table 1 collectively shows the evaluation results.

### (Film Condition)

The film surface on the substrate for evaluation was visually observed. If the surface was uniform with no discoloration, the film condition was rated as A; if the surface was uniform but with discoloration such as yellowing or blackening, the film condition was rated as B; and if the surface was nonuniform due to precipitation, volatilization, or the like, with discoloration, the film condition was rated as C.

### (Heat Resistance)

The film thickness of the substrate for evaluation was measured using a stylustype surface profiler (Dektak 150 manufactured by ULVAC, Inc.). The substrate for evaluation after the measurement was further heated on a hot plate at 300°C for 120 seconds and returned to room temperature, and then the film thickness was measured again. If the difference between the film thickness before the heating and that after the heating was less than 1%, the heat resistance was rated as A; and if the difference was 1% or greater, the heat resistance was rated as B.

### (Solvent Resistance)

The film thickness of the substrate for evaluation was measured using a stylustype surface profiler (Dektak 150 manufactured by ULVAC, Inc.). The substrate for evaluation after the measurement was immersed in PGMEA at 25°C for 60 seconds. PGMEA remaining on the film surface was removed by blowing air, followed by drying at 120°C for 60 seconds. Then, the substrate was returned to room temperature, and the film thickness was measured. If the difference between the film thickness before the immersion in the solvent and that after the immersion was less than 1%, the solvent resistance was rated as A; and if the difference was 1% or greater, the solvent resistance was rated as B.

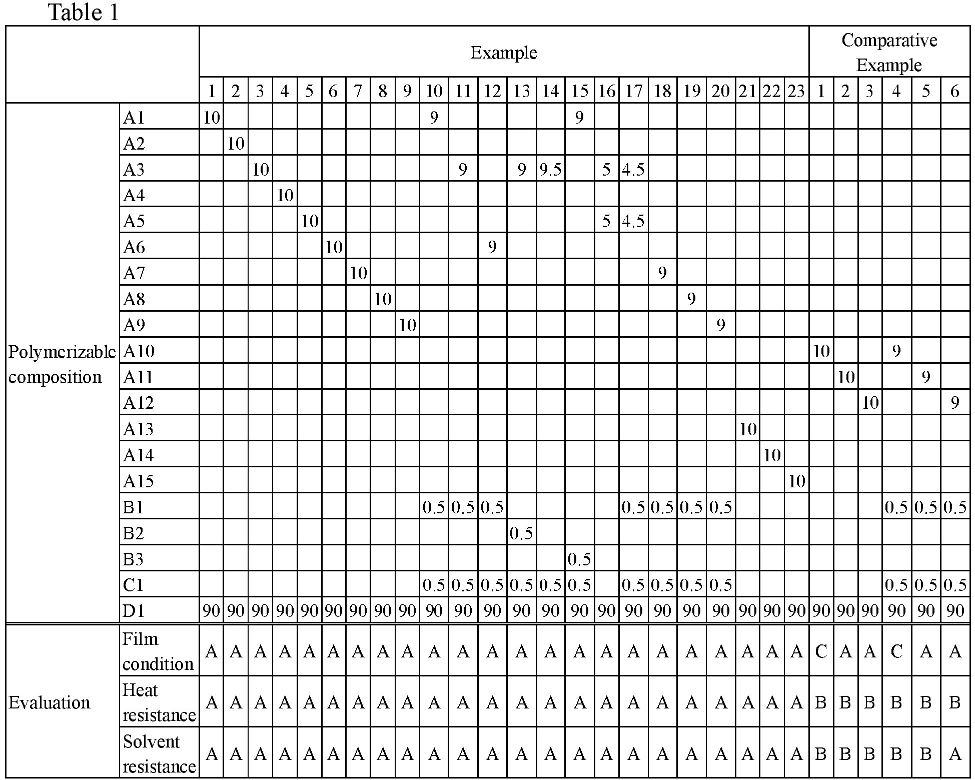

As shown in Table 1, the films obtained from the compositions containing the compounds of the present invention had uniform surfaces without discoloration and exhibited excellent heat resistance and excellent solvent resistance.

### Example 21 and Comparative Example 7

### (Preparation of Composition)

0.95 g of a test compound, 0.05 g of a photo-radical polymerization initiator (2-methyl-1-[4-(methylthio)phenyl]-2-morpholino-1-propanone), and 9.00 g of PGMEA were weighed, stirred, and dissolved. After it was confirmed that all solids were completely dissolved, the solution was filtered through a PTFE filter (pore size: 0.45 µm) to obtain a composition for evaluation.

The test compounds used were as follows.

| | | |
|---|---|---|
| Example 21 | S1: | Compound (97) |
| Comparative Example 7 | S2: | Compound (A10) |

### (Preparation of Substrate for Evaluation)

The prepared solution was applied to a glass substrate (EAGLE XG manufactured by Corning Incorporated) using a spin coater so as to achieve a film thickness of 1.0 µm after drying. The coated substrate was heated on a hot plate at 100°C for 120 seconds to be dried.

The heated and dried glass substrate was irradiated with UV rays (1000 mJ/cm²) using a high pressure mercury lamp.

### (Evaluation Results)

In the case of S1, which is a compound of the present invention, the film surface after the UV irradiation remained as transparent as it was after drying, and was harder than it was prior to the UV irradiation. In addition, no whitening or other changes were found even when the film surface was scrubbed with a nonwoven fabric soaked with acetone.

In the case of S2, the surface gradually whitened when the coated substrate after the UV irradiation was left to stand. In addition, when rubbed with a nonwoven fabric soaked with acetone, the film surface partially peeled off.

From the above results, it was clear that S1, which is the compound of the present invention, is UV curable.

### Synthesis of Compound (H14)

40.0 g (341 mmol) of indole, 47.2 g (341 mmol) of 2,5-dihydroxybenzaldehyde, and 243 g of acetonitrile were placed in a reaction flask equipped with a reflux tube and stirred until completely dissolved. After water-cooling, 5.7 g (34 mmol) of 48% hydrobromic acid was slowly added dropwise. After it was confirmed that heat generation had subsided, the mixture was returned to room temperature and then stirred for 2 hours. The reaction solution was cooled to 10°C, and the precipitate was collected by filtering and washed with 100 g of acetonitrile. The filter cake was dried under reduced pressure at 40°C to obtain compound (H14) as a gray powder.
Actual yield: 33.2 g (percent yield: 41%)
¹H-NMR (DMSO-d₆) δ/ppm: 6.00 (s, 2H), 6.04-7.33 (m, 14H), 8.27 (s, 2H), 9.25 (s, 2H), 10.42 (s, 2H)

### Synthesis of Compound (H8)

Then, 30.0 g (63.2 mmol) of the compound (H14), 19.3 g (75.9 mmol) of iodine, and 90 g of acetonitrile were placed in a reaction flask equipped with a reflux tube, the temperature was raised, and a reaction was carried out at 80°C for 14 hours. The reaction solution was cooled to room temperature, 30 g of a 10% aqueous sodium thiosulfate solution was added dropwise, and the mixture was cooled to 10°C under stirring. The precipitate was collected by filtering and washed with 50 g of acetonitrile, and the filter cake was dried under reduced pressure at 40°C to obtain compound (H8) as a pale brown powder.
Actual yield: 29.7 g (percent yield: 99%)
¹H-NMR (DMSO-d₆) δ/ppm: 6.80-7.43 (m, 14H), 8.60 (s, 2H), 8.89 (s, 2H), 10.27 (s, 2H)

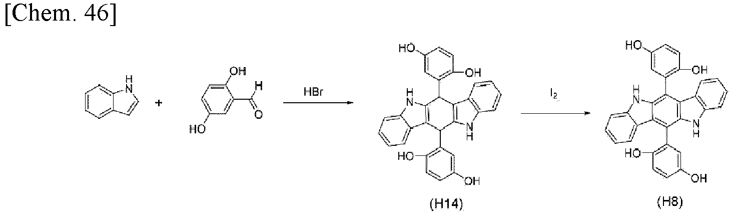

### Synthesis of Compound (H13)

Compound (H13) was obtained as a gray powder in the same manner as for the synthesis of the compound (H14), except that 3,4-dihydroxybenzaldehyde was used instead of 2,5-dihydroxybenzaldehyde.
Actual yield: 52.7 g (percent yield: 65%)
¹H-NMR (DMSO-d₆) δ/ppm: 5.44 (s, 2H), 6.49-7.26 (m, 14H), 8.63 (s, 4H), 10.55 (s, 2H)

### Synthesis of Compound (H7)

Compound (H7) was obtained as a pale yellow powder in the same manner as for the synthesis of the compound (H8), except that compound (H13) was used instead of compound (H14)..
Actual yield: 27.6 g (percent yield: 92%)
¹H-NMR (DMSO-d₆) δ/ppm: 6.85-7.43 (m, 14H), 9.10 (s, 2H), 9.22 (s, 2H), 10.39 (s, 2H)

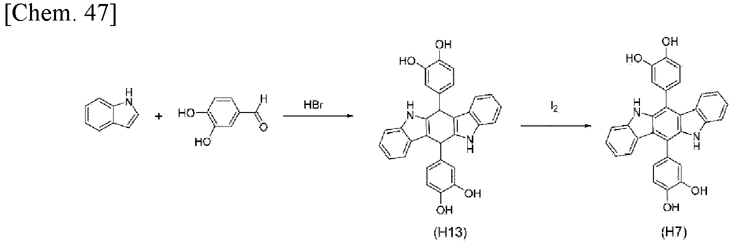

### Synthesis of Compound (H15)

Compound (H15) was obtained as a gray powder in the same manner as for the synthesis of compound (H14) were performed, except that 2,3-dihydroxybenzaldehyde was used instead of 2,5-dihydroxybenzaldehyde.
Actual yield: 47.0 g (percent yield: 58%)
¹H-NMR (DMSO-d₆) δ/ppm: 6.10 (s, 2H), 6.36-7.29 (m, 14H), 8.78 (s, 2H), 9.42 (s, 2H), 10.39 (s, 2H)

### Synthesis of Compound (H9)

Compound (H9) was obtained as a pale brown powder in the same manner as for the synthesis of compound (H8), except that compound (H15) was used instead of compound (H14).
Actual yield: 19.8 g (percent yield: 66%)
¹H-NMR (DMSO-d₆) δ/ppm: 6.79-7.44 (m, 14H), 8.21 (s, 2H), 9.48 (s, 2H), 10.22 (s, 2H)

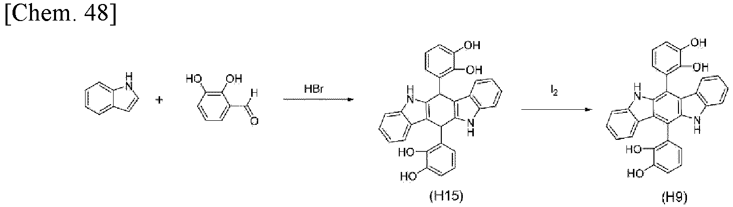

### Synthesis of Compound (H6)

29.3 g (250 mmol) of indole, 30.5 g (250 mmol) of 4-hydroxybenzaldehyde, and 166 g of acetonitrile were placed in a reaction flask equipped with a reflux tube and stirred until completely dissolved. After water-cooling, 4.2 g (25 mmol) of 48% hydrobromic acid was slowly added dropwise. After it was confirmed that heat generation had subsided, the mixture was returned to room temperature and then stirred for 2 hours. The reaction solution was cooled to 10°C. The precipitate was collected by filtering and washed with 70 g of acetonitrile, and the filter cake was dried under reduced pressure at 40°C to obtain compound (H6) as a gray powder.
Actual yield: 34.3 g (percent yield: 65%)
¹H-NMR (DMSO-d₆) δ/ppm: 5.52 (s, 2H), 6.64-7.23 (m, 16H), 9.17 (s, 2H), 10.56 (s, 2H)

### Synthesis of Compound (H3)

Then, 26.6 g (60 mmol) of compound (H6), 18.3 g (72 mmol) of iodine, and 80 g of acetonitrile were placed in a reaction flask equipped with a reflux tube, the temperature was raised, and a reaction was carried out at 80°C for 14 hours. The reaction solution was cooled to room temperature, 25 g of a 10% aqueous sodium thiosulfate solution was added dropwise, and the mixture was cooled to 10°C under stirring. The precipitate was collected by filtering and washed with 50 g of acetonitrile, and the filter cake was dried under reduced pressure at 40°C to obtain compound (H3) as a pale yellow powder.
Actual yield: 24.3 g (percent yield: 92%)
¹H-NMR (DMSO-d₆) δ/ppm: 6.81-7.47 (m, 16H), 9.67 (s, 2H), 10.40 (s, 2H)

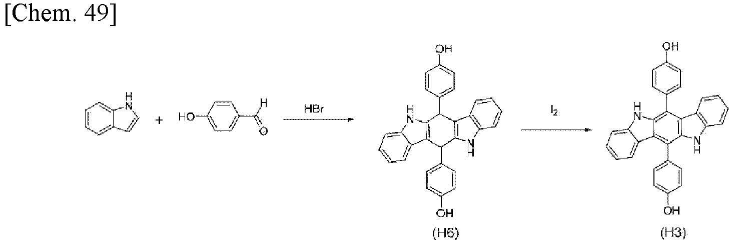

### Synthesis of Compound (H5)

Compound (H5) was obtained as a pale yellow powder in the same manner as for the synthesis of compound (H6), except that 3-hydroxybenzaldehyde was used instead of 4-hydroxybenzaldehyde.
Actual yield: 28.5 g (percent yield: 54%)
¹H-NMR (DMSO-d₆) δ/ppm: 5.57 (s, 2H), 6.59-7.25 (m, 16H), 9.16 (s, 2H), 10.66 (s, 2H)

### Synthesis of Compound (H2)

Compound (H2) was obtained as a pale yellow powder in the same manner as for the synthesis of compound (H3), except that compound (H5) was used instead of compound (H6).
Actual yield: 20.7 g (percent yield: 78%)
¹H-NMR (DMSO-d₆) δ/ppm: 6.83-7.49 (m, 16H), 9.68 (s, 2H), 10.49 (s, 2H)

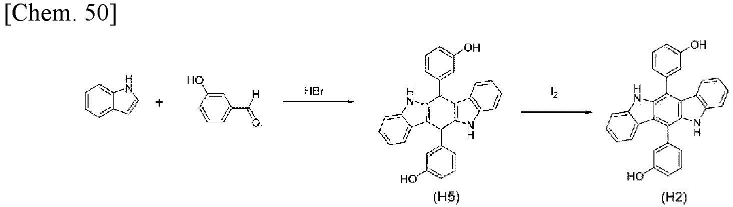

### Synthesis of Compound (H58)

39.4 g (300 mmol) of 1-methylindole, 51.7 g (300 mmol) of 2-hydroxy-1-naphthaldehyde, and 250 g of acetonitrile were placed in a reaction flask equipped with a reflux tube and stirred until completely dissolved. After water-cooling, 5.1 g (30 mmol) of 48% hydrobromic acid was slowly added dropwise. After it was confirmed that heat generation had subsided, the mixture was returned to room temperature and then stirred for 2 hours. The precipitate in the reaction solution was collected by filtering and washed with 100 g of acetonitrile, and the filter cake was dried under reduced pressure at 40°C to obtain compound (H58) as a pinkish gray powder.
Actual yield: 63.0 g (percent yield: 73.3%)
¹H-NMR (DMSO-d₆) δ/ppm: 3.67 (s, 6H), 6.84 (s, 2H), 7.06-8.34 (m, 20H), 9.89 (s, 2H)

### Synthesis of Compound (H57)

Then, 22.8 g (40.0 mmol) of compound (H58), 10.8 g (44.0 mmol) of chloranil, and 350 g of o-xylene were placed in a reaction flask equipped with a reflux tube, the temperature was raised, and a reaction was carried out at 140°C for 7 hours. The reaction solution was cooled to room temperature, and the solvent was removed therefrom. Then, 200 g of hexane and 100 g of isopropanol were added to the residue, and the mixture was stirred at room temperature for 30 minutes. The precipitate was collected by filtering and washed with 50 g of acetonitrile, and the filter cake was dried under reduced pressure at 40°C to obtain compound (H57) as a reddish brown powder.
Actual yield: 7.0 g (percent yield: 30.8%)
¹H-NMR (DMSO-d₆) δ/ppm: 3.84 (s, 6H), 7.08-8.25 (m, 20H), 9.67 (s, 2H)

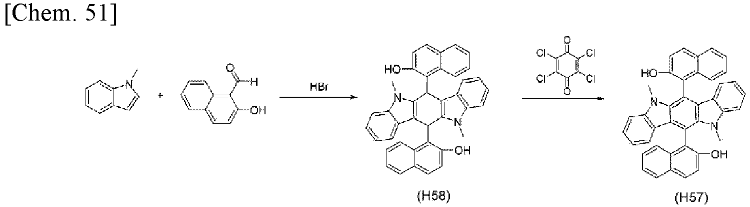

### Synthesis of Compound (H56)

1.31 g (10.0 mmol) of 1-methylindole, 1.72 g (10.0 mmol) of 4-hydroxy-1-naphthaldehyde, and 15.0 g of acetonitrile were placed in a reaction flask equipped with a reflux tube, and the liquid temperature was raised to 40°C under stirring. 0.17 g (1.0 mmol) of 48% hydrobromic acid was added dropwise, and the mixture was returned to room temperature and then stirred for 2 hours. The precipitate in the reaction solution was collected by filtering and washed with 10 g of acetonitrile, and the filter cake was dried under reduced pressure at 40°Cto obtain compound (H56) as a grayish purple powder.
Actual yield: 1.70 g (percent yield: 59.6%)
¹H-NMR (DMSO-d₆) δ/ppm: 3.97 (s, 6H), 4.09 (s, 2H), 6.50 (s, 2H), 6.70-8.61 (m, 20H)

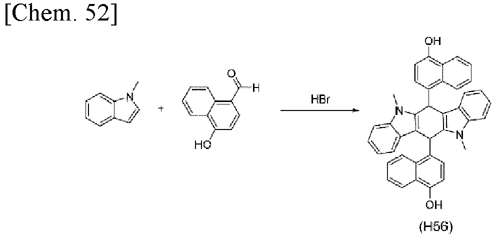

### Synthesis of Compound (H54)

1.36 g (11.6 mmol) of indole, 2.00 g (11.6 mmol) of 4-hydroxy-1-naphthaldehyde, and 15.0 g of acetonitrile were placed in a reaction flask equipped with a reflux tube, and the liquid temperature was raised to 40°C under stirring. 0.19 g (1.16 mmol) of 48% hydrobromic acid was added dropwise, and the mixture was returned to room temperature and then stirred for 2 hours. The precipitate in the reaction solution was collected by filtering and washed with 15 g of acetonitrile, and the filter cake was dried under reduced pressure at 40°C to obtain compound (H54) as a reddish purple powder.
Actual yield: 1.21 g (percent yield: 39.0%)
¹H-NMR (DMSO-d₆) δ/ppm: 4.53 (s, 2H), 6.48 (s, 2H), 6.71-8.19 (m, 20H), 10.75 (s, 2H)

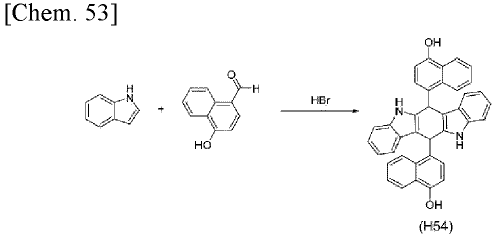

### Synthesis of Compound (H17)

3.80 g (32.4 mmol) of indole, 5.00 g (32.4 mmol) of 3,4,5-trihydroxybenzaldehyde, and 24.6 g of acetonitrile were placed in a reaction flask equipped with a reflux tube, and water-cooled under stirring. 1.09 g (6.5 mmol) of 48% hydrobromic acid was slowly added dropwise. After it was confirmed that heat generation had subsided, the mixture was returned to room temperature and then stirred for 2 hours. The precipitate in the reaction solution was collected by filtering and washed with 15 g of acetonitrile, and the filter cake was dried under reduced pressure at 40°C to obtain compound (H17) as a gray powder.
Actual yield: 2.48 g (percent yield: 15.0%)
¹H-NMR (DMSO-d₆) δ/ppm: 5.31 (s, 2H), 6.25 (s, 4H), 6.80-7.26 (m, 8H), 7.85 (s, 2H), 8.61 (s, 4H), 10.53 (s, 2H)

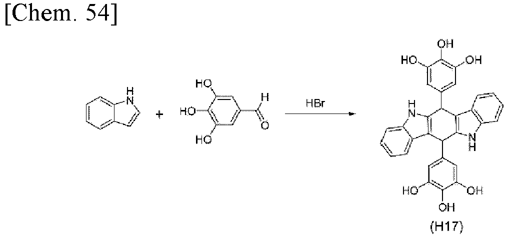

### Synthesis of Compound H71

14.0 g (71 mmol) of 5-bromoindole, 8.7 g (71 mmol) of 4-hydroxybenzaldehyde, and 64 g of acetonitrile were placed in a reaction flask equipped with a reflux tube and stirred until completely dissolved. After water-cooling, 1.2 g (7.1 mmol) of 48% hydrobromic acid was slowly added dropwise. After it was confirmed that heat generation had subsided, the mixture was returned to room temperature and then stirred for 2.5 hours. The reaction solution was cooled to 10°C. The precipitate was collected by filtering and washed with 22 g of acetonitrile, and the filter cake was dried under reduced pressure at 40°C to obtain compound (H71) as a pale yellow powder.
Actual yield: 8.8 g (percent yield: 41%)
¹H-NMR (DMSO-d₆) δ/ppm: 5.57 (s, 2H), 6.68-7.49 (m, 14H), 9.27 (s, 2H), 10.8 (s, 2H)

### Synthesis of Compound H72

Then, 4.0 g (6.66 mmol) of compound (H71), 2.0 g (8.00 mmol) of chloranil, and 19.9 g of dimethylformamide were placed in a reaction flask equipped with a reflux tube, the temperature was raised, and a reaction was carried out at 100°C for 2.5 hours. The reaction solution was cooled to room temperature, and the precipitate was collected by filtering, and then dispersed and washed with 10 g of methanol. Filtering was performed again, and the filter cake was washed with 15 g of methanol and then dried under reduced pressure at 60°C to obtain compound (B) as a pale yellow powder.
Actual yield: 3.2 g (percent yield: 80.2%)
¹H-NMR (DMSO-d₆) δ/ppm: 7.11-7.49 (m, 14H), 9.78 (s, 2H), 10.7 (s, 2H)

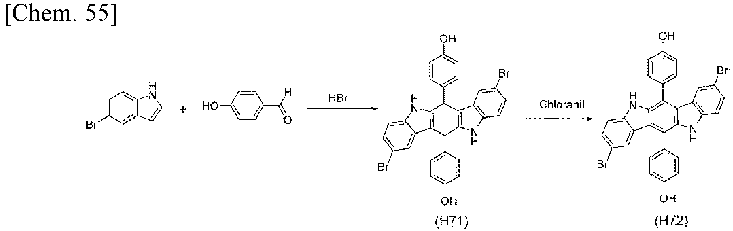

### Synthesis of Compound H81

4.0 g (30 mmol) of 5-hydroxyindole, 5.8 g (30 mmol) of 2-fluorenecarboxyaldehyde, and 30 g of acetonitrile were placed in a flask equipped with a reflux tube. 0.51 g (3 mmol) of 48% hydrobromic acid was added dropwise at room temperature under stirring, and the mixture was stirred for 2 hours. 10 g of water was added to the reaction solution, and the mixture was stirred at room temperature for 30 minutes and then filtered. The filter cake was washed three times with 10 g of methanol and then dried under reduced pressure at 40°C to obtain compound (H81) as a green powder.
Actual yield: 5.2 g (percent yield: 56%)
¹H-NMR (DMSO-d₆) δ/ppm: 4.12 (s, 4H), 5.66 (s, 2H), 6.13-8.29 (m, 22H), 10.09 (s, 2H), 10.33 (s, 2H)

### Synthesis of Compound H82

4.5 g (7 mmol) of compound (H81) and 90 g of acetonitrile were placed in a flask equipped with a reflux tube, and 2.2 g (9 mmol) of chloranil was added at room temperature under stirring. The mixture was stirred at room temperature for 30 minutes. Then, the temperature was raised to 70°C, and the mixture was heated and stirred at that temperature for 7 hours. The reaction solution was cooled to room temperature and then filtered. The filter cake was washed six times with 30 g of o-xylene and then dried under reduced pressure at 40°C to obtain compound (H82) as a gray powder.
Actual yield: 4.5 g (percent yield: 100%)
¹H-NMR (DMSO-d₆) δ/ppm: 4.12 (s, 4H), 6.43-8.24 (m, 22H), 10.09 (s, 2H), 10.33 (s, 2H)

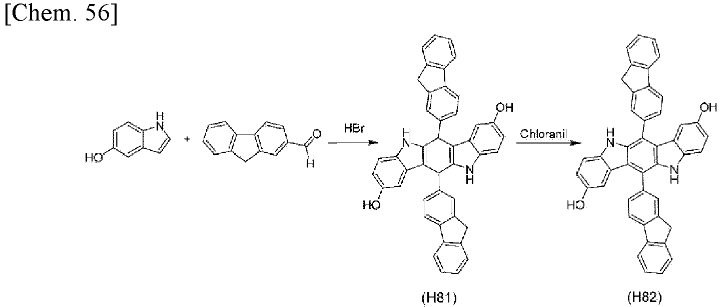

### Examples 24 to 37 and Comparative Examples 7 to 12

Components were weighed according to the formulation (parts by mass) shown in Table 2, and they were mixed with a solvent, and then dissolved through stirring. After it was confirmed that all solids were completely dissolved, the solution was filtered through a PTFE filter (pore size: 0.45 µm) to obtain a composition for evaluation.

The components shown in the table were as follows.

| | | | |
|---|---|---|---|
| A'1: | Compound (H2) | | Compound (I) |
| A'2: | Compound (H5) | | Compound (I) |
| A'3: | Compound (H7) | | Compound (I) |

| | | | |
|---|---|---|---|
| A'4: | Compound (H13) | Compound (I) | |
| A'5: | Compound (H8) | Compound (I) | |
| A'6: | Compound (H57) | Compound (I) | |
| A' 7: | Compound(A'7) below | | Phenolic compound, but not the compound (I) |
| A' 8: | Compound(A' 8) below | | Phenolic compound, but not the compound (I) |
| A' 9: | Compound (A' 9) below | | Compound with a specific skeleton, but without reactive group |
| B1: | Compound (B1) below | | Cross-linking agent (Glycoluril compound) |
| B2: | Compound (B2) below | | Cross-linking agent (Phenolic compound, but not the compound (I)) |
| B3: | Compound (B3) below | | Cross-linking agent (Phenolic compound, but not the compound (I)) |
| C1: | Bis(4-tert-butylphenyl)iodonium nonafluorobutanesulfonate (Polymerization initiator: Thermal acid generator) | | |
| D1: | Propylene glycol monomethyl ether acetate (PGMEA) solvent | | |

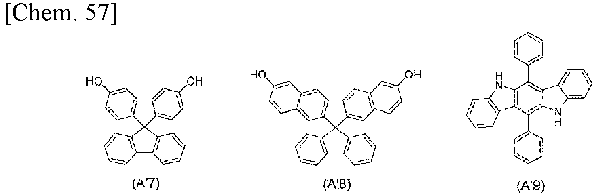

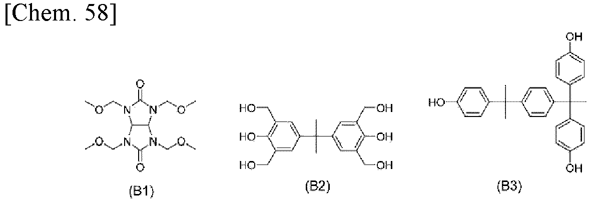

The prepared composition for evaluation was applied to a glass substrate (EAGLE XG manufactured by Corning Incorporated) using a spin coater so as to achieve a film thickness of 1.0 µm after heating. The coated substrate was heated on a hot plate at 170°C for 120 seconds and then further heated on a hot plate at 300°C for 120 seconds, to obtain a substrate for evaluation.

On the substrate for evaluation, the film condition, heat resistance, and solvent resistance were evaluated in the following manner. Table 2 collectively shows the evaluation results.

### (Film Condition)

The film surface on the substrate for evaluation was visually observed. If the surface was uniform with no discoloration, the film condition was rated as A; if the surface was uniform but with discoloration such as yellowing or blackening, the film condition was rated as B; and if unevenness due to precipitation, volatilization, or the like, and also discoloration were found, the film condition was rated as C.

A composition that forms a uniform surface with no discoloration can provide an excellent cured product and is thus preferred.

### (Heat Resistance)

The film thickness of the substrate for evaluation was measured using a stylustype surface profiler (Dektak 150 manufactured by ULVAC, Inc.). The substrate for evaluation after the measurement was further heated on a hot plate at 300°C for 120 seconds and returned to room temperature, and then the film thickness was measured again. If the difference between the film thickness before the heating and that after the heating was less than 5%, the heat resistance was rated as A; and if the difference was 5% or greater, the heat resistance was rated as B.

A small difference in the film thickness after heating means high heat resistance and is thus preferred.

### (Solvent Resistance)

The film thickness of the substrate for evaluation was measured using a stylustype surface profiler (Dektak 150 manufactured by ULVAC, Inc.). The substrate for evaluation after the measurement was immersed in PGMEA at 25°C for 60 seconds. PGMEA remaining on the film surface was removed by blowing air, followed by drying at 120°C for 60 seconds. Then, the substrate was returned to room temperature, and the film thickness was measured. If the difference between the film thickness before the immersion in the solvent and that after the immersion was less than 1%, the solvent resistance was rated as A; and if the difference was 1% or greater, the solvent resistance was rated as B.

Excellent solvent resistance means adaptability to a wide range of applications and is therefore preferred.

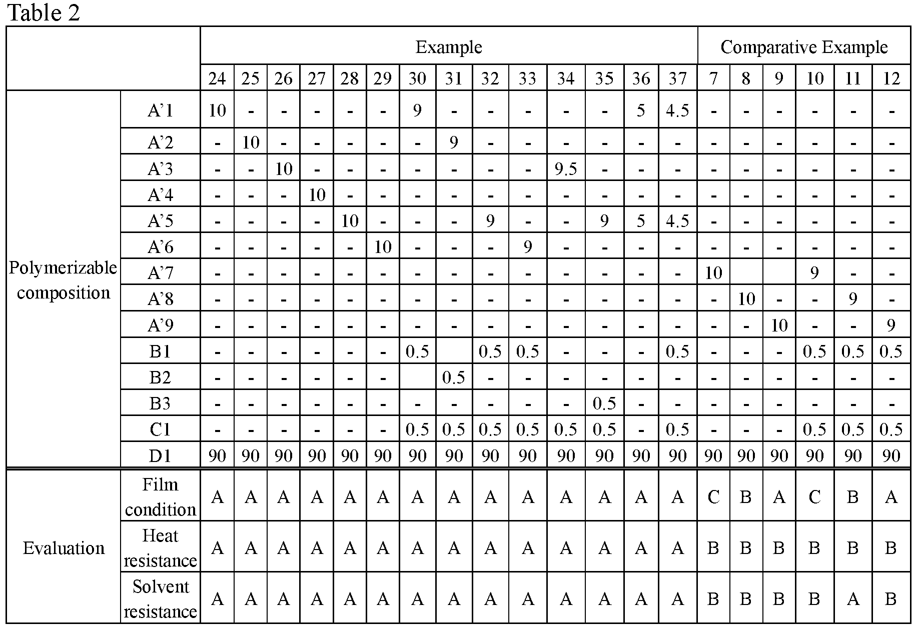

As shown in Table 1, the films obtained from the compositions containing the compounds of the present invention had uniform surfaces without discoloration and exhibited excellent heat resistance and excellent solvent resistance.

### Industrial Applicability

A composition containing a compound of the present invention gives a cured product having excellent heat resistance and excellent solvent resistance. Therefore, the composition is useful as a composition for electronic component applications that require high heat resistance.

## Claims

1. A compound represented by the general formula (I) below and having at least one reactive group in a molecule: 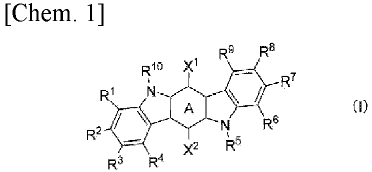
where A represents a hydrocarbon ring having 6 carbon atoms;
X¹ and X² each independently represent an aryl group having 6 to 30 carbon atoms and optionally substituted with a reactive group or a group having a reactive group, a heterocyclic group having 2 to 30 carbon atoms and optionally substituted with a reactive group or a group having a reactive group, or a heterocycle-containing group having 3 to 30 carbon atoms and optionally substituted with a reactive group or a group having a reactive group;
R¹, R², R³, R⁴, R⁶, R⁷, R⁸, and R⁹ each independently represent a hydrogen atom, a halogen atom, a reactive group, a nitro group, an aliphatic hydrocarbon group having 1 to 20 carbon atoms and optionally substituted with a reactive group, an aromatic hydrocarbon ring-containing group having 6 to 20 carbon atoms and optionally substituted with a reactive group, a heterocyclic group having 2 to 10 carbon atoms and optionally substituted with a reactive group, or a heterocycle-containing group having 3 to 30 carbon atoms and optionally substituted with a reactive group, or a group formed by replacing at least one methylene group in the aliphatic hydrocarbon group having 1 to 20 carbon atoms by a divalent group selected from <group A> below, a group formed by replacing at least one methylene group in the aromatic hydrocarbon ring-containing group having 6 to 20 carbon atoms by a divalent group selected from <group A> below, or a group formed by replacing at least one methylene group in the heterocycle-containing group having 3 to 30 carbon atoms by a divalent group selected from <group A> below; and
R⁵ and R¹⁰ each independently represent a hydrogen atom, an aliphatic hydrocarbon group having 1 to 20 carbon atoms and optionally substituted with a reactive group, an aromatic hydrocarbon ring-containing group having 6 to 20 carbon atoms and optionally substituted with a reactive group, a heterocyclic group having 2 to 10 carbon atoms and optionally substituted with a reactive group, or a heterocycle-containing group having 3 to 30 carbon atoms and optionally substituted with a reactive group, or a group formed by replacing at least one methylene group in the aliphatic hydrocarbon group having 1 to 20 carbon atoms by a divalent group selected from <group A> below, a group formed by replacing at least one methylene group in the aromatic hydrocarbon ring-containing group having 6 to 20 carbon atoms by a divalent group selected from <group A> below, or a group formed by replacing at least one methylene group in the heterocycle-containing group having 3 to 30 carbon atoms by a divalent group selected from <group A> below:
<group A>: -O-, -CO-, -COO-, -OCO-, -NR¹¹-, -NR¹²CO-, -S-
where R¹¹ and R¹² each independently represent a hydrogen atom or a hydrocarbon group having 1 to 20 carbon atoms, and
with a proviso that the reactive group is a group selected from a carbon-carbon double bond, a carbon-carbon triple bond, a nitrile group, an epoxy group, an isocyanate group, a hydroxy group, a phenolic hydroxy group, an amino group, and a thiol group.

2. The compound according to claim 1, wherein the reactive group is a group other than a phenolic hydroxy group.

3. The compound according to claim 2, wherein X¹ and X² each represent a phenyl group optionally substituted with a reactive group or a group having a reactive group, a hydrocarbon-type aromatic fused ring group having 7 to 30 carbon atoms and optionally substituted with a reactive group or a group having a reactive group, or a heterocycle-containing fused ring group having 3 to 30 carbon atoms and optionally substituted with a reactive group or a group having a reactive group.

4. The compound according to claim 2, wherein X¹ and X² each represent a phenyl group optionally substituted with a reactive group or a group having a reactive group, a naphthyl group optionally substituted with a reactive group or a group having a reactive group, an anthracenyl group optionally substituted with a reactive group or a group having a reactive group, a fluorenyl group optionally substituted with a reactive group or a group having a reactive group, a carbazolyl group optionally substituted with a reactive group or a group having a reactive group, or a pyrenyl group optionally substituted with a reactive group or a group having a reactive group.

5. The compound according to any one of claims 2 to 4, wherein R⁵ and R¹⁰ each represent a group having a reactive group.

6. The compound according to any one of claims 2 to 5, wherein the reactive group is a carbon-carbon triple bond.

7. The compound according to any one of claims 2 to 6, wherein the compound has three or more reactive groups in the molecule.

8. The compound according to claim 1,
wherein the reactive group is a phenolic hydroxy group;
X¹ and X² each independently represent an aryl group having 6 to 30 carbon atoms and optionally substituted with a phenolic hydroxy group, a heterocyclic group having 2 to 30 carbon atoms and optionally substituted with a phenolic hydroxy group, or a heterocycle-containing group having 3 to 30 carbon atoms and optionally substituted with a phenolic hydroxy group;
R¹, R², R³, R⁴, R⁶, R⁷, R⁸, and R⁹ each independently represent a hydrogen atom, a halogen atom, a phenolic hydroxy group, a nitro group, an aliphatic hydrocarbon group having 1 to 20 carbon atoms, an aromatic hydrocarbon ring-containing group having 6 to 20 carbon atoms and optionally substituted with a phenolic hydroxy group, a heterocyclic group having 2 to 10 carbon atoms and optionally substituted with a phenolic hydroxy group, a heterocycle-containing group having 3 to 30 carbon atoms and optionally substituted with a phenolic hydroxy group, or a group formed by replacing at least one methylene group in the aliphatic hydrocarbon group having 1 to 20 carbon atoms by a divalent group selected from <group A>, a group formed by replacing at least one methylene group in the aromatic hydrocarbon ring-containing group having 6 to 20 carbon atoms by a divalent group selected from <group A>, or a group formed by replacing at least one methylene group in the heterocycle-containing group having 3 to 30 carbon atoms by a divalent group selected from <group A>; and
R⁵ and R¹⁰ each independently represent a hydrogen atom, an aliphatic hydrocarbon group having 1 to 20 carbon atoms, an aromatic hydrocarbon ring-containing group having 6 to 20 carbon atoms and optionally substituted with a phenolic hydroxy group, a heterocyclic group having 2 to 10 carbon atoms and optionally substituted with a phenolic hydroxy group, or a heterocycle-containing group having 3 to 30 carbon atoms and optionally substituted with a phenolic hydroxy group, or a group formed by replacing at least one methylene group in the aliphatic hydrocarbon group having 1 to 20 carbon atoms by a divalent group selected from <group A>, a group formed by replacing at least one methylene group in the aromatic hydrocarbon ring-containing group having 6 to 20 carbon atoms by a divalent group selected from <group A>, or a group formed by replacing at least one methylene group in the heterocycle-containing group having 3 to 30 carbon atoms by a divalent group selected from <group A>.

9. The compound according to claim 8, wherein X¹ and X² each represent a group selected from the group consisting of a phenyl group, a naphthyl group, an anthracenyl group, and a pyrenyl group each having a phenolic hydroxy group.

10. The compound according to claim 8 or 9, wherein the compound has two or more phenolic hydroxy groups in the molecule.

11. The compound according to any one of claims 1 to 10, wherein the compound is represented by the general formula (Ia), (Ib), or (Ic) below: 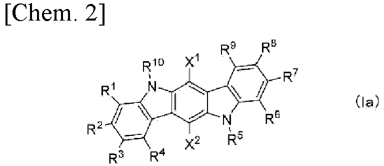
wherein symbols in the formula are as defined in the general formula (I); 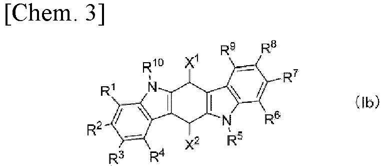
wherein symbols in the formula are as defined in the general formula (I); and 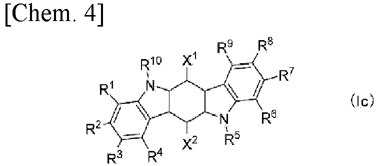
wherein symbols in the formula are as defined in the general formula (I).

12. A composition comprising the compound according to any one of claims 1 to 11.

13. The composition according to claim 12, comprising a cross-linking agent.

14. The composition according to claim 12 or 13, comprising a polymerization initiator.

15. A composition for an electronic component, comprising the composition according to any one of claims 12 to 14.

16. A cured product of the composition according to any one of claims 12 to 14.
